(19) Europäisches Patentamt
European Patent Office
Office européen des brevets

(11) EP 4 015 629 A1

(12) EUROPEAN PATENT APPLICATION

(43) Date of publication:
22.06.2022 Bulletin 2022/25

(21) Application number: 20215594.1

(22) Date of filing: 18.12.2020

(51) International Patent Classification (IPC):
*C12N 9/96* (2006.01)

(52) Cooperative Patent Classification (CPC):
C11D 3/2093; C11D 3/3788; C11D 3/38663;
C12N 9/14; C12N 9/96

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: BASF SE
67056 Ludwigshafen am Rhein (DE)

(72) Inventors:
- HUEFFER, Stephan
67056 Ludwigshafen (DE)
- WEISS, Thomas
67056 Ludwigshafen (DE)
- ETTL, Roland
67056 Ludwigshafen (DE)
- SPECKER ,Daniel
67056 Ludwigshafen (DE)
- BARTULI, Larissa
67056 Ludwigshafen (DE)

(74) Representative: BASF IP Association
BASF SE
GBI-C006
67056 Ludwigshafen (DE)

(54) **POLYMER MIXTURES FOR INCREASING STABILITY AND PERFORMANCE OF HYDROLASE-CONTAINING DETERGENTS**

(57) Mixture comprising at least
component (a): graft polymer comprising as a graft base a polyether and as grafted side chains copolymers comprising at least one comonomer selected from

$CH_2$=CH-O-C(O)-$R^3$ (Ia)

$CH_2$=CH-$CH_2$-O-C(O)-$R^3$ (Ib)

$CH_2$=CZ-CO-$OR^4$ (Ic)

wherein $R^3$ is selected from $C_1$-$C_{21}$-alkyl, for example methyl, n-propyl, n-pentyl, n-heptyl, n-nonyl, iso-nonyl, n-undecyl, n-tridecyl, n-pentadecyl, n-heptadecyl, or n-nonadecyl.
$R^4$ is selected from $C_2$-$C_{20}$-alkyl, preferably with an even number of carbon atoms, for example ethyl, n- and iso propyl, n-hexyl, isohexyl, sec-hexyl, n-heptyl, n-octyl, 2-ethylhexyl, n-nonyl, n-decyl or isodecyl, n-$C_{12}H_{25}$, n-$C_{14}H_{29}$, n-$C_{16}H_{33}$ or n-$C_{18}H_{37}$,
and Z is selected from hydrogen and methyl, hydrogen bring preferred

component (b): Trialkyl citrate according to formula (TAC):

EP 4 015 629 A1

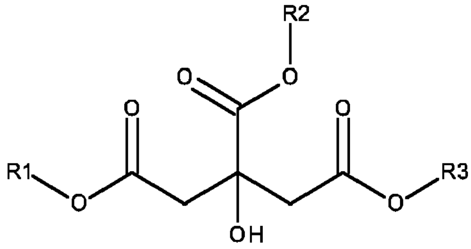

(TAC)

wherein the variables in formula (TAC) are defined as follows:
R1, R2, R3 are selected from H, linear C1-C8 alkyl, and branched C3-C8 alkyl, wherein at least one of R1, R2, and R3 is not H. Examples of linear C1-C8 alkyl are methyl, ethyl, n-propyl, n-butyl, n-pentyl, etc.
Examples of branched C3-C8 alkyl are 2-propyl, 2-butyl, sec.-butyl, tert.-butyl, 2-pentyl, 3-pentyl, iso-pentyl, etc. Preferably, R1, R2, R3 are ethyl.

SEQUENCE LISTING

<110> BASF SE

<120> Polymer mixtures for increasing stability and performance of hydrolase-containing detergents

<130> 202798EP01

<140> 20215594.1
<141> 18 December 2020

<160> 5

<170> PatentIn version 3.5

<210> 1
<211> 7
<212> PRT
<213> Artificial

<220>
<223> Motif SEQ ID NO: 25 of WO 2019/081724

<220>
<221> MISC_FEATURE
<222> (1)..(1)
<223> Xaa = Asp (D) or Met (M) or Leu (L)

<220>
<221> MISC_FEATURE
<222> (2)..(2)
<223> Xaa = Ser (S) or Thr (T)

<220>
<221> MISC_FEATURE
<222> (7)..(7)
<223> Xaa = Asp (D) or Asn (N)

<400> 25

Xaa Xaa Gly Tyr Ser Arg Xaa
1               5

<210> 2
<211> 8
<212> PRT
<213> Artificial

<220>
<223> Motif SEQ ID NO: 26 of WO 2019/081724

<220>
<221> MISC_FEATURE
<222> (3)..(3)
<223> Xaa = any amino acid

<400> 26

Ala Ser Xaa Asn Arg Ser Lys Gly

1 5

<210> 3
<211> 4
<212> PRT
<213> Artificial Sequence

<220>
<223> Motif SEQ ID NO: 73 of WO 2017/060493

<220>
<221> MISC_FEATURE
<222> (1)..(1)
<223> Xaa = Gly (G) or Tyr (Y) or Trp (W) or Phe (F) or Ala (A) or His (H)

<220>
<221> MISC_FEATURE
<222> (4)..(4)
<223> Xaa =Arg (R) or Gln (Q) or Asp (D) or Glu (E) or Val (V)

<400> 73

Xaa Asn Ile Xaa
1

<210> 4
<211> 5
<212> PRT
<213> Artificial Sequence

<220>
<223> Motif SEQ ID NO: 74 of WO 2017/060493

<220>
<221> MISC_FEATURE
<222> (4)..(4)
<223> Xaa = Asp (D) or His(H) or Leu (L)

<400> 74

Ser Asp His Xaa Pro
1 5

<210> 5
<211> 5
<212> PRT
<213> Artificial Sequence

<220>
<223> Motif SEQ ID NO: 73 of WO 2017/060493

<220>
<221> MISC_FEATURE
<222> (5)..(5)
<223> Xaa = Arg (R) or Gln (Q)

<400> 75

Gly Gly Asn Ile Xaa
1 5

## Description

The invention in one aspect relates to a mixture comprising (a) at least one graft polymer (GP) comprising as a graft base a polyether and (b) at least one trialkylcitrate. In another aspect, the invention relates to the use of said mixture as a detergent component in enzyme containing detergent formulations.

Graft polymer mixture (GPM)

The inventive mixtures herein may be called graft polymer mixtures (GPM).

component (a) - grafted polymer

Mixtures according to the invention comprise as component (a) at least one graft polymer, hereinafter also in brief "graft polymer (GP)", comprising as a graft base a polyether and as grafted side chains copolymers comprising at least one comonomer (CM) selected from

$CH_2$=CH-O-C(O)-$R^3$ (CM-Ia)

$CH_2$=CH-$CH_2$-O-C(O)-$R^3$ (CM-Ib)

$CH_2$=CZ-CO-O$R^4$ (CM-Ic)

wherein $R^3$ is selected from $C_1$-$C_{21}$-alkyl, for example methyl, n-propyl, n-pentyl, n-heptyl, n-nonyl, iso-nonyl, n-undecyl, n-tridecyl, n-pentadecyl, n-heptadecyl, or n-nonadecyl.
$R^4$ is selected from $C_2$-$C_{20}$-alkyl, preferably with an even number of carbon atoms, for example ethyl, n- and iso propyl, n-hexyl, isohexyl, sec-hexyl, n-heptyl, n-octyl, 2-ethylhexyl, n-nonyl, n-decyl or isodecyl, n-$C_{12}H_{25}$, n-$C_{14}H_{29}$, n-$C_{16}H_{33}$ or n-$C_{18}H_{37}$, and Z is selected from hydrogen and methyl, hydrogen bring preferred.

In the context of the present invention, the polyethers bear at least 5 ether groups per mole and - if at all - only hydroxyl groups, for example one, two or three hydroxyl groups per molecule. Such hydroxyl groups may be primary or secondary hydroxyl groups, primary hydroxyl groups being preferred.

In one embodiment, polyethers are polyethylene glycols, for example with an average molecular weight $M_n$ in the range of from 500 to 25,000 g/mole, preferably 1,000 to 15,000 g/mole and even more preferably 1,500 to 10,000 g/mol. In one embodiment, polyethylene glycols have an average molecular weight $M_n$ in the range of 1,500 to 4,000 g/mole. In one embodiment, polyethylene glycols have an average molecular weight $M_n$ in the range of 4,000 to 6,000 g/mole. In one embodiment, polyethylene glycols have an average molecular weight $M_n$ in the range of 5,000 to 8,000 g/mole.

In one embodiment, polyethers are polypropylene glycols, for example with an average molecular weight $M_n$ in the range of from 500 to 20,000 g/mole, preferably 2,000 to 10,000 g/mole and even more preferably 4,000 to 9,000 g/mol.

In one embodiment, polyethers are copolymers of ethylene glycol and propylene glycol units, for example random copolymers and preferably block copolymers, for example di-block copolymers and tri-block copolymers. Preferably, copolymers of ethylene glycol and propylene glycol are block copolymers (also called EO-PO block copolymer herein), preferably di-block copolymers, wherein the ratio of ethylene glycol to propylene glycol is from about 1:1 to 2:3. In one embodiment, the EO-PO block copolymers with a ratio ethylene glycol:propylene glycol of about 1:1 have an average molecular weight $M_n$ in the range of 4,000 to 10,000 g/mole, more preferably from 4,500 to 9,500 g/mole. In one embodiment, the EO-PO block copolymers with a ratio ethylene glycol:propylene glycol of about 2:3 have an average molecular weight $M_n$ in the range of 2,000 to 6,000 g/mole, more preferably from 2,900 to 5,800 g/mole.

In one embodiment of the present invention, the graft base ofGP is selected from polyethylene glycols, polypropylene glycols and EO-PO block copolymers, each non-capped or end-capped with $C_1$-$C_{20}$-alkyl or $C_3$-$C_{20}$-2-hydroxyalkyl

Polyethers in the context of the present invention are preferably non-capped. In such embodiments, polyethers may also be referred to as polyether polyols, and they have terminal hydroxyl groups. Polyethers are preferably selected from polyethylene glycols, polypropylene glycols, and from copolymers of ethylene glycol and propylene glycol.

Examples of $C_1$-$C_{20}$-alkyl are methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl or isodecyl, n-$C_{12}H_{25}$, n-$C_{14}H_{29}$, n-$C_{16}H_{33}$ or n-$C_{18}H_{37}$, preferred are $C_1$-$C_4$-alkyl, for example methyl,

ethyl, n-propyl, n-butyl, and in particular methyl.

Examples of $C_3$-$C_{20}$-2-hydroxyalkyl are 2-hydroxy-n propyl, 2-hydroxy-n-hexyl, 2-hydroxy-n-octyl, 2-hydroxy-n-decyl, 2-hydroxy-n-dodecyl, 2-hydroxy-n-tetradecyl, 2-hydroxy-n-hexadecyl, 2-hydroxy-n-octadecyl, and 2-hydroxy-n-eicosyl.

GP according to the invention preferably comprise side chains in copolymerized form preferably selected from comonomers of general formulae (CM-Ia), (CM-Ib) and (CM-Ic) as described above.

Specific examples of comonomers according to formula (CM-Ia)are vinylacetate, vinylpropionate, vinylbutyrate, vinyl-n-hexanoate, vinyl-n-octanoate, vinyl-2-ethylhexanoate, vinyllaurate, vinylstearate, vinylmyristate, and vinylpalmitate.

Specific examples of comonomers according to formula (CM-Ib) are allylpropionate, allylbutyrate, allyl-n-hexanoate, allyl-n-octanoate, allyl-2-ethylhexanoate, allyllaurate, vinylstearate, allylmyristate, and allylpalmitate.

Specific examples of comonomers according to formula (CM-Ic) are 2-ethylhexyl(meth)acrylate, 2-n-propylheptyl(meth)acrylate, stearyl(meth)acrylate, lauryl(meth)acrylate, palmityl(meth)acrylate, and myristyl(meth)acrylate.

GP according to the invention preferably comprises side chains in copolymerized form preferably selected from comonomers of general formula (CM-Ia)and (CM-Ic).

Preferred comonomers according to formula (CM-Ia)are vinylacetate, vinylpropionate, vinylbutyrate, vinyl-2-ethylhexanoate, and vinyl laurate.

Preferred comonomers according to formula (CM-Ic) are 2-ethylhexylacrylate and lauryl(meth)acrylate.

In one embodiment, the GP comprises as a graft base a polyether and as grafted side chains copolymers comprising at least two comonomers selected from (CM-Ia), (CM-Ib), and (CM-Ic) as disclosed above.

Preferably, the GP comprises as a graft base a polyether and as grafted side chains copolymers comprising at least two comonomers selected from (CM-Ia) and (CM-Ic) as disclosed above. Preferably, the GP comprises one comonomer selected from (Ia) and one comonomer selected from (CM-Ic), preferably wherein the comonomer (CM-Ia) is vinylacetate and the comonomer (CM-Ic) is lauryl(meth)acrylate. In one embodiment, the weight ratio (CM-Ia):(CM-Ic), preferably vinylacetate:lauryl(meth)acrylate, is 7:3 or 8:2 or 9:1, preferably 8:2.

In one embodiment of the present invention, GP have a weight ratio of graft base to side chains in copolymerized form in the range of from 9:1 to 1:2, preferably 5:1 to 1:2 and even more preferably 4:1 to 1:1.

In one embodiment of the present invention, GP have an average molecular weight $M_n$ in the range of from 2,250 to 200,000 g/mol, preferred are 2,250 to 25,000 g/mol, even more preferred are 2,500 to 10,000 g/mol. The average molecular weight $M_n$ may be determined by gel permeation chromatography (GPC), with polyethylene glycol as comparison standard. The grafting as such may be confirmed by HPLC (High Pressure Liquid Chromatography).

In one embodiment of the present invention, GP have a broad weight distribution. A broad weight distribution in the context of GP according to the invention means that such GP have a polydispersity $Q = M_w/M_n$ of at least 2.5, preferably from 3.5 to 10, more preferably from 4 to 8.

In another embodiment of the present invention, GP have a narrow weight distribution. A narrow weight distribution in the context of GP according to the invention means that such GP have a polydispersity $Q = M_w/M_n$ of at most 3.25, preferably from 1.5 to 3.0, more preferably from 2 to 2.5.

Suitable as GP for the present invention are also those grafted polymers as disclosed in WO 0018375, when for the preparation of the graft polymers with polyether (being the "graft base" in this present invention) and only vinyl esters (as "comonomers" within this present invention) are employed and those vinyl esters such as vinyl acetate are not hydrolyzed after graft polymerization.

component (b) - trialkyl citrate

Mixtures according to the invention comprise as component (b) at least one trialkyl citrate according to formula (TAC):

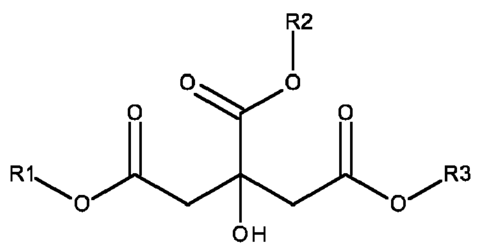

(TAC)

wherein the variables in formula (TAC) are defined as follows:

R1, R2, R3 are selected from H, linear C1-C8 alkyl, and branched C3-C8 alkyl, wherein at least one of R1, R2, and R3 is not H. Examples of linear C1-C8 alkyl are methyl, ethyl, n-propyl, n-butyl, n-pentyl, etc. Examples of branched C3-C8 alkyl are 2-propyl, 2-butyl, sec.-butyl, tert.-butyl, 2-pentyl, 3-pentyl, iso-pentyl, etc. Preferably, R1, R2, R3 are ethyl.

Graft polymer mixtures of the invention are resulting from adding at least one trialkyl citrate according to formula (TAC) as described above

I. during the grafting process of GP;

II. before or during the step of removal of volatile components from GP or

III. during or after the cooling step following the removal of volatile components.

The weight ratio of GP:trialkyl citrate in the resulting GPM is within the range of 10:1 to 1:1. In one embodiment, the weight ration GP:trialkyl citrate in the GPM is 5:1 to 1:1, 3:1 to 1:1, or 2:1 to 1:1.

The GPM of the invention is preferably liquid at 20°C and 101.3 kPa. Liquid in this context means that the GPM is pourable even if being highly viscous. In one embodiment, viscosity is below 20000 mPa*s, below 10000 mPa*s or below 5000 mPa*s at 25°C.

In one embodiment, the GPM is of white or liqht yellow color. Whiteness means the degree to which a surface is white. White is usually void of hue and grayness. Hue may be defined technically in the CIECAM02 model as "the degree to which a stimulus can be described as similar to or different from stimuli that are described as red, orange, yellow, green, blue, purple." Grayness may correspond to shade which is a pure color mixed with black (or having a lower lightness). In the HSV model colors correspond to certain values of hue-saturation-brightness. Off-white corresponds to hue 0°, saturation 0%, brightness 100%. Liqht yellow color preferably has a brightness of about 85%-100%, hue angle may be about 60°.

The GPM of the invention is preferably free from water. Free from water means that essentially no water is comprised in the GPM. Specifically this means that water is comprised in amounts less than 10% by weight, less than 8% by weight, less than 5% by weight, less than 4% by weight, less than 3% by weight, less than 2% by weight, or less than 1% by weight.

Preparation of GPM

In general polyether as disclosed above is placed into a reaction container; at a certain temperature a feed of radical starter is commenced and is to be continued during the time of addition of the comonomer(s) (CM as disclosed above); at or shortly after start of the addition of radical starter, at least one comonomer is added over a certain period of time; after complete addition of the comonomer, addition of radical starter is continued for a further period of time. After completion of addition of the radical starter, the reaction mixture is kept at the polymerization temperature and /or at a temperature higher than the polymerization temperature for a further period of time.

The volatiles contained within the reaction mixture are removed by suitable means to obtain a polymer solution.

At least one trialkyl citrate according to formula (TAC) as described above is added

I. during the grafting process of GP;

II. before or during the step of removal of volatile components from GP or

III. during or after the cooling step following the removal of volatile components.

In one embodiment, GPM of the invention is prepared by the steps of

i. heating at least one polyether as disclosed above to about 50-120°C, preferably about 70-100°C, more preferably about 80-95°C, such as about 90°C under $N_2$-atmosphere, at reduced, ambient or increased pressure, preferably ambient pressure

ii. feeding radical starter (suitable for the chosen reaction conditions) over a time period of about 3-10h, preferably about 5-9h, more preferably about 6-8h, such as about 7h (feed 1)

iii. 0-30 min, preferably 5-20 min, such as 15 min +/-2 min after feed 1 commenced, feeding at least one comonomer (CM) as disclosed above over a time period of about 3-8h, preferably about 4-7h, more preferably about 5-6h (feed 2)

iv. after completion of feed 2, feeding radical starter over a time period of about 0.5-5h, preferably about 1-4h, more preferably about 2-3h (feed 3)

v. after completion of feed 3, stirring at a temperature in the range of 100°C to 130°C for a time period of about 60min to 90 min

vi. reducing pressure to 10 to 40 mbar for removal of volatile components

vii. cooling the reaction mixture,

wherein at least one trialkyl citrate is added during or directly after step (ii), during or directly after step (iii), during or directly after step (v.) or during or directly after step (vii.).

In one embodiment, addition of at least one trialkyl citrate takes place during step (vii.) at the latest, before the mixture is cooled down to a temperature of about 50°C to 90°C, or to a temperature of about 50°C to 70°C. Preferably, at least one trialkyl citrate is added before the mixture is cooled down to a temperature of about 70°C, of about 75°C, of about 80°C, or of about 85°C. When addition of at least one trialkyl citrate takes place during step (vii.), the mixture is stirred at said temperature for at least 10 min before it is cooled down to room temperature.

The term "about x°C" is intended to mean here that the temperature variations are preferably kept within the range of +/- 10°C, more preferably in the range of +/- 5°C.

The term "about xh" herein means that the time variation is preferably kept within the range +/- 10 min, more preferably in the range of +/-5 min.

In one embodiment, the amount of CM introduced in step iii corresponds to a weight ratio of polyether (provided in step i):comonomer (CM) in the range of 10:1 to 1:2, preferably 9:1 to 1:1, more preferably 9:1 to 2:1.

According to the invention, radical starter, polyether and comonomer (CM) are advantageously added in such a way that a low and substantially constant concentration of undecomposed radical starter, polyether and comonomer (CM) is present in the reaction mixture. The proportion of undecomposed radical starter in the overall reaction mixture is preferably $\leq$ 15% by weight, in particular $\leq$ 10% by weight, based on the total amount of radical starter metered in during the comonomer addition.

Depending on the mean polymerization temperature, examples of particularly suitable radical starters are:

- at a mean polymerization temperature of from 50 to 60°C:
  tert-butyl peroxyneoheptanoate, tert-butyl peroxyneodecanoate, tert-amyl peroxypivalate, tert-amyl peroxyneodecanoate, 1,1,3,3-tetramethylbutyl peroxyneodecanoate, cumyl peroxyneodecanoate, 1,3-di(2-neodecanoyl peroxyisopropyl)benzene, di(n-butyl) peroxydicarbonate and di(2-ethylhexyl) peroxydicarbonate;

- at a mean polymerization temperature of from 60 to 70°C:
  tert-butyl peroxypivalate, tert-butyl peroxyneoheptanoate, tert-butyl peroxyneodecanoate, tert-amyl peroxypivalate and di(2,4-dichlorobenzoyl) peroxide;

- at a mean polymerization temperature of from 70 to 80°C:
  tert-butyl peroxypivalate, tert-butyl peroxyneoheptanoate, tert-amyl peroxypivalate, dipropionyl peroxide, dicapryloyl peroxide, didecanoyl peroxide, dilauroyl peroxide, di(2,4-dichlorobenzoyl) peroxide and 2,5-dimethyl-2,5-di(2-ethylhexanoylperoxy)hexane;

- at a mean polymerization temperature of from 80 to 90°C:
  tert-butyl peroxyisobutyrate, tert-butyl peroxy-2-ethylhexanoate, tert-amyl peroxy-2-ethylhexanoate, dipropionyl peroxide, dicapryloyl peroxide, didecanoyl peroxide, dilauroyl peroxide, di(3,5,5-trimethylhexanoyl) peroxide, dibenzoyl peroxide and di(4-methylbenzoyl) peroxide;

- at a mean polymerization temperature of from 90 to 100°C:
  tert-butyl peroxyisobutyrate, tert-butyl peroxy-2-ethylhexanoate, tert-butyl monoperoxymaleate, tert-amyl peroxy-2-ethylhexanoate, dibenzoyl peroxide and di(4-methylbenzoyl) peroxide;

- at a mean polymerization temperature of from 100 to 110°C:
  tert-butyl monoperoxymaleate, tert-butyl peroxyisobutyrate and tert-amyl peroxy(2-ethylhexyl)carbonate;

- at a mean polymerization temperature of from 110 to 120°C:
  tert-butyl monoperoxymaleate, tert-butyl peroxy-3,5,5-trimethylhexanoate and tert-amyl peroxy(2-ethylhexyl)carbonate.

In one embodiment, the radical starter added in step ii. istert.-butyl-peroctoate.
The total amount of radical starter used in the process is preferably from 0.01 to 5% by weight, preferably from 0.01 to 3.5% by weight, more preferably 0.01 to 1% by weight, relative in each case on the total weight of comonomers (CM) added in step iii.
Preferably, 40-70% of radical starter are added in step ii, and the rest is added in step iv.

Graft polymer formulation (GPF)

In one aspect, the invention relates to a graft polymer formulation (GPF) comprising GPM and at least one surfactant selected from non-ionic surfactants (NIS) and amphoteric surfactants (AS).
The method to prepare the GPF of the invention comprises the step of adding at least one surfactant selected from non-ionic surfactants (NIS) and amphoteric surfactants (AS) to the GPM of the invention. Preferably, at least one surfactant as disclosed above is added to the GPM of the invention at a temperature above the melting point of the surfactant. If the melting point of the surfactant is higher than ambient temperature, the surfactant is added during the cooling phase at a point in time where the GPM has a temperature above the melting temperature of the surfactant. The temperature of the GPM however has to be chosen in a way that the surfactant is not degraded at this temperature.
In one aspect, the GPF of the invention comprises one or more NIS. The GPF may comprise one NIS or mixtures of at least two NIS. Mixtures of NIS include mixtures of NIS of one type according to a general formula as disclosed below and mixtures of more types according to general formulae as disclosed below.
In one embodiment, the GPF comprises at least one non-ionic surfactant selected from selected from compounds of general formula (NIS-I):

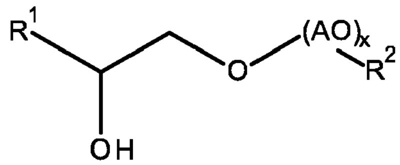

(NIS-I)

The variables of the general formula (NIS-I) are defined as follows:

AO being identical or different alkylene oxides, selected from $CH_2$-$CH_2$-O, $(CH_2)_3$-O, $(CH_2)_4$-O, $CH_2CH(CH_3)$-O, $CH(CH_3)$-$CH_2$-O- and $CH_2CH(n$-$C_3H_7)$-O.

$R^1$ is selected from linear (straight-chain; n-) or branched $C_3$-$C_{30}$-alkyl, and from straight-chain or branched $C_4$-$C_{30}$-alkylene with at least one C-C double bond. In one embodiment, $R^1$ is selected from straight-chain or branched $C_3$-$C_{17}$-alkyl, preferably from n-$C_4$-$C_{12}$-alkyl.

$R^2$ is selected from linear (straight-chain; n-) or branched $C_1$-$C_{30}$-alkyl, and from straight-chain or branched $C_2$-$C_{30}$-alkylene with at least one C-C double bond. $R^2$ may be straight-chain or branched $C_6$-$C_{20}$-alkyl, preferably straight-chain or branched $C_8$-$C_{12}$-alkyl, more preferably straight-chain or branched $C_{10}$-$C_{12}$-alkyl.

The integer x of the general formula (NIS-I) may be a number in the range of 5 to 70, 10 to 60, 15 to 50, or 20 to 40.
In one embodiment of the present invention, $(AO)_x$ is selected from $(CH_2CH_2O)_{x1}$, x1 being selected from one to 50.
In one embodiment of the present invention, $(AO)_x$ is selected from $-(CH_2CH_2O)_{x2}-(CH_2CH(CH_3)-O)_{x3}$ and $-(CH_2CH_2O)_{x2}-(CH(CH_3)CH_2-O)_{x3}$, x2 and x3 being identical or different and selected from 1 to 30.
In one embodiment of the present invention, $(AO)_x$ is selected from $-(CH_2CH_2O)_{x4}$, x4 = being in the range of from 10 to 50, AO being EO, and $R^1$ and $R^2$ each being independently selected from $C_8$-$C_{14}$-alkyl.
In the context of the present invention, x or x1 or x2 and x3 or x4 are to be understood as average values, the number average being preferred. Therefore, each x or x1 or x2 or x3 or x4 - if applicable - can refer to a fraction although a specific molecule can only carry a whole number of alkylene oxide units.
In one embodiment, GPF of the invention comprise at least one non-ionic surfactant according to formula (NIS-I), wherein $R^1$ is n-$C_3$-$C_{17}$ alkyl, $R^2$ is linear or branched $C_8$-$C_{14}$ alkyl. Preferably AO is selected from $-(CH_2CH_2O)_{x2}-(CH_2CH(CH_3)-O)_{x3}$, $-(CH_2CH_2O)_{x2}-(CH(CH_3)CH_2-O)_{x3}$ , and $-(CH_2CH_2O)_{x4}$, wherein x2 and x4 is a

number in the range of 15-50 and x3 is a number in the range of 1 to 15. At least one non-ionic surfactant in one embodiment is a compound according to formula (NIS-I), wherein $R^1$ is n-$C_8$ alkyl, $R^2$ is branched $C_{11}$ alkyl, AO is $CH_2$-$CH_2$-O, and x is 22.

At least one non-ionic surfactant in one embodiment is a compound according to formula (NIS-I), wherein $R^1$ is n-$C_8$-$C_{10}$ alkyl, $R^2$ is n-$C_8$-$C_{10}$ alkyl, AO is $CH_2$-$CH_2$-O, and x is 40.

At least one non-ionic surfactant in one embodiment is a compound according to formula (NIS-I), wherein $R^1$ is n-$C_8$ alkyl, $R^2$ is n-$C_{10}$ alkyl, AO is selected from $-(CH_2CH_2O)_{x2}-(CH_2CH(CH_3)-O)_{x3}$, $-(CH_2CH_2O)_{x2}-(CH(CH_3)CH_2-O)_{x3}$ , wherein x2 = 22 and x3 = 1.

At least one non-ionic surfactant in one embodiment is a compound according to formula (NIS-I), wherein $R^1$ is n-$C_5$ alkyl, $R^2$ is n-$C_{12}$ alkyl, AO is selected from $-CH_2CH_2O$, and x is 32.

In one embodiment, at least one non-ionic surfactant is selected from compounds of the general formula (NIS-II), which might be called alkyl-polyglycosides (APG):

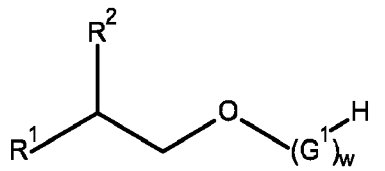

(NIS-II)

The variables of the general formula (NIS-II) are defined as follows:

$R^1$ is selected from $C_1$-$C_{17}$ alkyl and $C_2$-$C_{17}$ alkenyl, wherein alkyl and/or alkenyl are linear (straight-chain; n-) or branched; examples are n-$C_5H_{11}$, n-$C_6H_{13}$, n-$C_7H_{15}$, n-$C_9H_{19}$, n-$C_{11}H_{23}$, n-$C_{13}H_{27}$, n-$C_{15}H_{31}$, n-$C_{17}H_{35}$, i-$C_9H_{19}$, i-$C_{12}H_{25}$.

$R^2$ is selected from H, $C_1$-$C_{17}$ alkyl and $C_2$-$C_{17}$ alkenyl, wherein alkyl and/or alkenyl are linear (straight-chain; n-) or branched.

$G^1$ is selected from monosaccharides with 4 to 6 carbon atoms, such as glucose and xylose.

The integer w of the general formula (NIS-II) is in the range of from 1.1 to 4, w being an average number.

In an embodiment, GPF comprises at least one non-ionic surfactant selected from compounds according to formulae (NIS-I) and (NIS-II), both as disclosed above. In a preferred embodiment, GPF comprise at least one NIS according to general formula NIS-I as disclosed above.

In one embodiment, at least one non-ionic surfactant is selected from compounds of general formula (NIS-III):

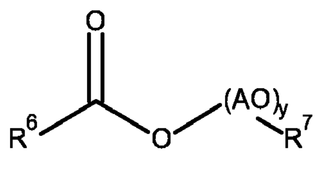

(NIS-III)

The variables of the general formula (NIS-III) are defined as follows:

AO is selected from ethylene oxide (EO), propylene oxide (PO), butylene oxide (BO), and mixtures thereof.

$R^6$ is selected from $C_5$-$C_{17}$ alkyl and $C_5$-$C_{17}$ alkenyl, wherein alkyl and/or alkenyl are linear (straight-chain; n-) or branched.

$R^7$ is selected from H, $C_1$-$C_{18}$-alkyl, wherein alkyl is linear (straight-chain; n-) or branched.

The integer y of the general formula (III) is a number in the range of 1 to 70, preferably 7 to 15.

Non-ionic surfactants may further be selected from sorbitan esters and/or ethoxylated or propoxylated sorbitan esters. Non-limiting examples are products sold under the trade names SPAN and TWEEN.

Non-ionic surfactants may further be selected from alkoxylated mono- or di-alkylamines, fatty acid monoethanolamides (FAMA), fatty acid diethanolamides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), and combinations thereof.

In one embodiment, the GPF comprises at least one amphoteric surfactant selected from compounds comprising amphoteric structures of general formula (AS-I), which might be called modified amino acids (proteinogenic as well as non-proteinogenic):

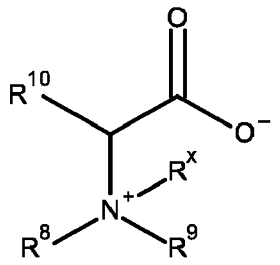

(AS-I)

The variables in general formula (AS-I) are defined as follows:

$R^8$ is selected from H, $C_1$-$C_4$ alkyl, $C_2$-$C_4$ alkenyl, wherein alkyl and/or are linear (straight-chain; n-) or branched.

$R^9$ is selected from $C_1$-$C_{22}$-alkyl, $C_2$-$C_{22}$-alkenyl, $C_{10}$-$C_{22}$alkylcarbonyl, and $C_{10}$-$C_{22}$ alkenylcarbonyl.

$R^{10}$ is selected from H, methyl, $-(CH_2)_3NHC(NH)NH_2$, $-CH_2C(O)NH_2$, $-CH_2C(O)OH$, - $(CH_2)_2C(O)NH_2$, $-(CH_2)_2C(O)OH$, (imidazole-4-yl)-methyl, $-CH(CH_3)C_2H_5$, $-CH_2CH(CH_3)_2$, - $(CH_2)_4NH_2$, benzyl, hydroxymethyl, $-CH(OH)CH_3$, (indole-3-yl)-methyl, (4-hydroxy-phenyl)-methyl, isopropyl, $-(CH_2)_2SCH_3$, and $-CH_2SH$.

$R^x$ is selected from H and $C_1$-$C_4$-alkyl.

In one embodiment, the GPF comprises at least one amphoteric surfactant selected from compounds comprising amphoteric structures of general formulae (AS-IIa), (AS-IIb), or (AS-IIc), which might be called betaines and/or sulfobetaines:

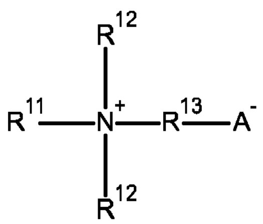

(AS-IIa)

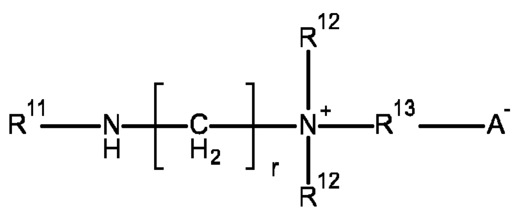

(AS-IIb)

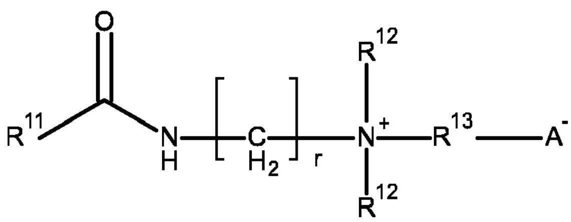

(AS-IIc)

The variables in general formulae (AS-IIa), (AS-IIb) and (AS-IIc) are defined as follows:

$R^{11}$ is selected from linear (straight-chain; n-) or branched $C_7$-$C_{22}$ alkyl and linear (straight-chain; n-) or branched $C_7$-$C_{22}$ alkenyl.

$R^{12}$ are each independently selected from linear (straight-chain; n-) $C_1$-$C_4$ alkyl.

$R^{13}$ is selected from $C_1$-$C_5$ alkyl and hydroxy $C_1$-$C_5$ alkyl; for example 2-hydroxypropyl.

$A^-$ is selected from carboxylate and sulfonate.

The integer r in general formulae (AS-IIa), (AS-IIb), and (AS-IIc) is in the range of 2 to 6.

In one embodiment, the GPF comprises at least one amphoteric surfactant selected from compounds comprising amphoteric structures of general formula (AS-III), which might be called alkyl-amphocarboxylates:

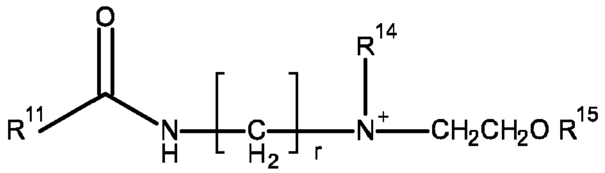

(AS-III)

The variables in general formula (AS-III) are defined as follows:

$R^{11}$ is selected from $C_7$-$C_{22}$ alkyl and $C_7$-$C_{22}$ alkenyl, wherein alkyl and/or alkenyl are linear (straight-chain; n-) or branched, preferably linear.

$R^{14}$ is selected from $-CH_2C(O)O^-M^+$, $-CH_2CH_2C(O)O^-M^+$ and $-CH_2CH(OH)CH_2SO_3^-M^+$.

$R^{15}$ is selected from H and $-CH_2C(O)O^-$

The integer r in general formula (AS-III) is in the range of 2 to 6.

Non-limiting examples of further suitable alkyl-amphocarboxylates include sodium cocoamphoacetate, sodium lauroamphoacetate, sodium capryloamphoacetate, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, and disodium capryloamphodipropionate.

In one embodiment, the GPF comprises at least one amphoteric surfactant selected from compounds comprising amphoteric structures of general formula (AS-IV), which might be called amine oxides (AO):

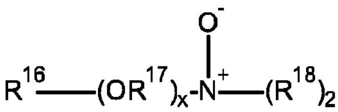

(AS-IV)

The variables in general formula (AS-IV) are defined as follows:

$R^{16}$ is selected from $C_8$-$C_{18}$ alkyl, hydroxy $C_8$-$C_{18}$ alkyl, acylamidopropoyl and $C_8$-$C_{18}$ alkyl phenyl group; wherein alkyl and/or alkenyl are linear (straight-chain; n-) or branched.

$R^{17}$ is selected from $C_2$-$C_3$ alkylene, hydroxy $C_2$-$C_3$ alkylene, and mixtures thereof.

$R^{18}$: each residue can be independently selected from $C_1$-$C_3$ alkyl and hydroxy $C_1$-$C_3$; $R^{15}$ groups can be attached to each other, e.g., through an oxygen or nitrogen atom, to form a ring structure.

The integer x in general formula (AS-IV) is in the range of 0 to 5, preferably from 0 to 3, most preferably 0.

Non-limiting examples of further suitable amine oxides include $C_{10}$-$C_{18}$ alkyl dimethyl amine oxides and $C_8$-$C_{18}$ alkoxy ethyl dihydroxyethyl amine oxides. Examples of such materials include dimethyloctyl amine oxide, diethyldecyl amine oxide, bis-(2-hydroxyethyl)dodecyl amine oxide, dimethyldodecylamine oxide, dipropyltetradecyl amine oxide, methylethylhexadecyl amine oxide, dodecylamidopropyl dimethyl amine oxide, cetyl dimethyl amine oxide, stearyl dimethyl amine oxide, tallow dimethyl amine oxide and dimethyl-2-hydroxyoctadecyl amine oxide.

A further example of a suitable amine oxide is cocamidylpropyl dimethylaminoxide, sometimes also called cocamidopropylamine oxide.

Mixtures of two or more different amphoteric surfactants may be present in in a GPF according to the present

invention.

Preferably, GPF of the invention comprise at least one amphoteric surfactant selected from compounds according to formula (AS-IV) as described above.

In one embodiment, the GPF comprises at least one NIS and at least one AS as disclosed above.

In one embodiment, the GPF comprises

(a) at least one NIS selected from compounds according to general formulae NIS-I, NIS-II and NIS-III, preferably at least one compound according to general formula NIS-I, and/or

(b) at least one AS selected from compounds according to general formulae AS-I, AS-II, AS-III and AS-IV

and at least one NIS according to general formulae NIS-IVa or NIS-IVb:

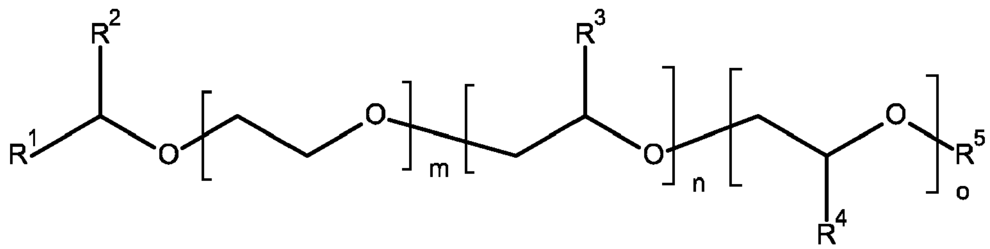

(NIS-IVa)

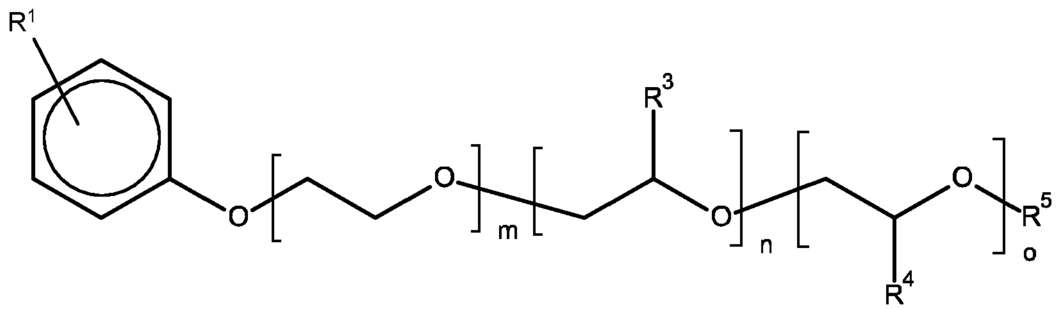

(NIS-IVb)

The variables of the general formulae (NIS-IVa) and (NIS-IVb) are defined as follows:

$R^1$ is selected from $C_1$-$C_{23}$ alkyl and $C_2$-$C_{23}$ alkenyl, wherein alkyl and/or alkenyl are linear (straight-chain; n-) or branched; examples are n-$C_5H_{11}$, n-$C_6H_{13}$,n-$C_7H_{15}$, n-$C_9H_{19}$, n-$C_{11}H_{23}$, n-$C_{13}H_{27}$, n-$C_{15}H_{31}$, n-$C_{17}H_{35}$, i-$C_9H_{19}$, i-$C_{12}H_{25}$.

$R^2$ is selected from H, $C_1$-$C_{20}$ alkyl and $C_2$-$C_{20}$ alkenyl, wherein alkyl and/or alkenyl are linear (straight-chain; n-) or branched.

$R^3$ and $R^4$, each independently selected from $C_1$-$C_{16}$ alkyl, wherein alkyl is linear (straight-chain; n-) or branched; examples are methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl, isodecyl.

$R^5$ is selected from H and $C_1$-$C_{18}$ alkyl, wherein alkyl is linear (straight-chain; n-) or branched.

The integers of the general formulae (NIS-IVa) and (NIS-IVb) are defined as follows:
m is in the range of zero to 200, preferably 1-80, more preferably 3-20; n and o, each independently in the range of zero to 100; n preferably is in the range of 1 to 10, more preferably 1 to 6; o preferably is in the range of 1 to 50, more preferably 4 to 25. The sum of m, n and o is at least one, preferably the sum of m, n and o is in the range of 5 to 100, more preferably in the range of from 9 to 50.

The non-ionic surfactants of the general formulae (NIS-IVa) and (NIS-IVb) may be of any structure, is it block or random structure, and is not limited to the displayed sequence of formulae (NIS-IVa) and (NIS-IVb).

Compounds according to formula (NIS-IVa) may be called alkyl polyethyleneglycol ether (AEO) herein. Compounds according to formula (NIS-IVb) may be called alkylphenol polyethyleneglycol ether (APEO) herein.
In one embodiment, NIS-IVa is characterized in $R^1$ being n-$C_{13}H_{27}$, $R^2$ and $R^5$ being H, m being 3-20, n and o = 0.
In one embodiment, NIS-IVa is characterized in R1 being linear or branched $C_{10}$ alkyl, $R^2$ and $R^5$ being H, m being 3-14, n and o = 0.
In one embodiment, NIS-IV is a mixture of two non-ionic surfactants according to said general formula NIS-IVa, wherein one of said non-ionic surfactants is characterized in $R^1$ being n-$C_{15}H_{31}$, $R^2$ and $R^5$ being H, m being 11-80, n and o = 0, and the other surfactant is characterized in $R^1$ being n-$C_{17}$ $H_{35}$, $R^2$ and $R^5$ being H, m being 11-80, n and o = 0.
In one embodiment, NIS-IVa is characterized in m being in the range of 3 to 11, preferably not more than 10, more preferably not more than 7; n and o being 0, $R^1$ being linear $C_9$-$C_{17}$ alkyl, $R^2$ and $R^5$ being H.
In one embodiment, NIS-IV is a mixture of two non-ionic surfactants according to said general formula NIS-IVa, wherein one of said non-ionic surfactants is characterized in $R^1$ being n-$C_{12}H_{25}$, $R^2$ and $R^5$ being H, m being 3-30, preferably 7, n and o = 0, and the other surfactant is characterized in $R^1$ being n-$C_{14}H_{29}$, $R^2$ and $R^5$ being H, m being 3-30, preferably 7, n and o = 0.
In one embodiment, NIS-IV is a mixture of two non-ionic surfactants according to said general formula NIS-IVa, wherein one of said non-ionic surfactants is characterized in $R^1$ being n-$C_{11}$ $H_{23}$, $R^2$ and $R^5$ being H, m being 4-10, n and o = 0, and the other surfactant is characterized in $R^1$ selected from n-$C_{11}H_{23}$ and n-$C_{17}H_{35}$, $R^2$ and $R^5$ being H, m being 4-10, n and o = 0.
In one embodiment, NIS-IV is a mixture of two non-ionic surfactants according to said general formula NIS-IVa, wherein one of said non-ionic surfactants is characterized in $R^1$ being n-$C_9H_{19}$, $R^2$ and $R^5$ being H, m being 5-7, n and o = 0, and the other surfactant is characterized in $R^1$ being n-$C_{17}H_{35}$, $R^2$ and $R^5$ being H, m being 5-7, n and o = 0.
In one embodiment, NIS-IV is a mixture of two non-ionic surfactants according to said general formula NIS-IVa, wherein one of said non-ionic surfactants is characterized in $R^1$ being n-$C_{11}H_{23}$, $R^5$ being H, m being 7, n and o = 0, and the other surfactant is characterized in $R^1$ being $C_{13}H_{27}$, $R^5$ being H, m being 7, n and o = 0.

## Detergent formulation

The present invention is directed to detergent formulations that are useful for cleaning laundry and dishware. Further, the invention is directed to a process for making such detergent formulations.
Detergent formulations of the invention are preferably liquid at 20°C and 101.3 kPa and comprise at least one GPM of the invention and one or more detergent components.
Preferably, detergent formulations of the invention comprise at least
component (a) as disclosed herein is comprised in amounts in the range of 0.1% to 10% by weight, preferably 0.25% to 5% by weight and more preferably 0.5% to 2.5% weight and
component (b) as disclosed herein is comprised in amounts in the range of from 0.05 to 5% by weight, preferably 0.2% to 2.5% by weight and more preferably 0.4% to 1% by weight,
all relative to the total weight of the detergent formulation.
In one embodiment, detergent formulations of the invention comprise 0.25% to 10% by weight, preferably 0.5% to 5% by weight and more preferably 1% to 3% by weight GPM of the invention.
In one embodiment, detergent formulations of the invention comprise 0.25% to 10% by weight, preferably 0.5% to 5% by weight and more preferably 1% to 3% by weight GPF of the invention.
In one embodiment of the present invention, inventive detergent formulations comprise

- 0.25% to 10% by weight, preferably 0.5% to 5% by weight and more preferably 1% to 3% by weight GPM or GPF of the invention
- in the range of from 0.05 to 5% by weight, preferably 0.1 to 5% by weight and more preferably 0.2 to 4% weight of at least one hydrolase (EC 3),
- in the range of from 1 to 10 % by weight, preferably 2 to 8 % by weight and more preferably up to 5 % by weight of surfactant, and, optionally,
- in the range of from 1 to 50% by weight, preferably 10 to 40 % by weight of complexing agent,

all relative to the total weight of the detergent formulation.
In one embodiment, inventive detergent formulations comprise at least one hydrolase (EC 3). Preferred enzymes are selected from the group of enzymes acting on ester bond (E.C. 3.1), glycosylases (E.C. 3.2), and peptidases (E.C. 3.4). Enzymes acting on ester bond (E.C. 3.1), are hereinafter also referred to as lipases, and DNAses. Glycosylases

(E.C. 3.2) are hereinafter also referred to as either amylases, cellulases, or mannanases. Peptidases (E.C. 3.4) are hereinafter also referred to as proteases.

Hydrolases usually are identified by polypeptide sequences (also called amino acid sequences herein). The polypeptide sequence specifies the three-dimensional structure including the "active site" of an enzyme which in turn determines the catalytic activity of the same. Polypeptide sequences may be identified by a SEQ ID NO. According to the World Intellectual Property Office (WIPO) Standard ST.25 (1998) the amino acids herein are represented using three-letter code with the first letter as a capital or the corresponding one letter.

Any enzyme comprised in detergent formulations of the invention according to the invention relates to

- a parent polypeptide (sequence) or amino acid sequence and/or
- a variant parent polypeptide (sequence) or amino acid sequence,

both having enzymatic activity. Proteins or polypeptides having enzymatic activity are enzymatically active or exert enzymatic conversion, meaning that enzymes act on substrates and convert these into products. The term "enzyme" herein excludes inactive variants of an enzyme.

The term parent enzyme (or parent sequence) includes wild-type enzymes (sequences) and synthetically generated sequences (enzymes) which are used as starting sequences for introduction of (further) changes. The term "enzyme variant" or "sequence variant" or "variant enzyme" refers to an enzyme that differs from its parent enzyme in its amino acid sequence to a certain extent. If not indicated otherwise, variant enzyme "having enzymatic activity" means that this variant enzyme has the same type of enzymatic activity as the respective parent enzyme.

In describing enzyme variants usually substitutions, deletions and insertions occur when compared to a parent sequence. Herein nomenclature is used known to those skilled in the art. Amino acid substitutions are usually described by providing the original amino acid followed by the number of the position within the amino acid sequence, followed by the substituted amino acid. Amino acid deletions are usually described by providing the original amino acid followed by the number of the position within the amino acid sequence, followed by *. Amino acid insertions are usually described by providing the original amino acid followed by the number of the position within the amino acid sequence, followed by the original amino acid and the additional amino acid. Where different alterations can be introduced at a position, the different alterations are separated by a comma.

Enzyme variants are usually defined by their sequence identity when compared to a parent enzyme. Sequence identity usually is provided as "% sequence identity" or "% identity". For calculation of sequence identities, in a first step a sequence alignment has to be produced. According to this invention, a pairwise global alignment has to be produced, meaning that two sequences have to be aligned over their complete length, which is usually produced by using a mathematical approach, called alignment algorithm.

According to the invention, the alignment is generated by using the algorithm of Needleman and Wunsch (J. Mol. Biol. (1979) 48, p. 443-453). Preferably, the program "NEEDLE" (The European Molecular Biology Open Software Suite (EMBOSS)) is used for the purposes of the current invention, with using the programs default parameter (gap open=10.0, gap extend=0.5 and matrix=EBLOSUM62).

According to this invention, the following calculation of %-identity applies: %-identity = (identical residues / length of the alignment region which is showing the respective sequence of this invention over its complete length) *100.

According to this invention, enzyme variants are described as an amino acid sequence which is at least n% identical to the amino acid sequence of the respective parent enzyme with "n" being an integer between 10 and 100. In one embodiment, variant enzymes are at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical when compared to the full-length amino acid sequence of the parent enzyme, wherein the enzyme variant has enzymatic activity.

"Enzymatic activity" means the catalytic effect exerted by an enzyme, which usually is expressed as units per milligram of enzyme (specific activity) which relates to molecules of substrate transformed per minute per molecule of enzyme (molecular activity).

In one embodiment, detergent formulations of the invention comprise at least one hydrolase comprising as a catalytic triad the amino acids aspartate, histidine and serine. In one embodiment, at least one hydrolase comprising a catalytic triad has a motif selected from aspartate-histidine-serine and serine-histidine-aspartate, wherein the relative order of these amino acids reads from the amino to carboxy-terminus.

In a preferred embodiment, the hydrolase comprises a catalytic triad having the motif serine-histidine-aspartate, wherein the relative order of these amino acids reads from the amino to carboxy-terminus. Preferably, said hydrolase is selected from proteases and lipases.

In one embodiment, at least one protease is selected from the group consisting of serine endopeptidases (EC 3.4.21), most preferably chymotrypsin related proteases (3.4.21.1) and subtilisin type proteases (EC 3.4.21.62).

In one embodiment, at least one protease comprises a catalytic triad having a motif selected from aspartate-histidine-serine and serine-histidine-aspartate, wherein the relative order of these amino acids reads from the amino to carboxy-terminus.

Subtilisins and chymotrypsin related serine proteases both have a catalytic triad comprising aspartate, histidine and serine. In the subtilisin related proteases the relative order of these amino acids, reading from the amino to carboxy-terminus is aspartate-histidine-serine. In the chymotrypsin related proteases the relative order, however is histidine-aspartate-serine.

Preferably, at least one protease comprises a catalytic triad comprising the amino acids aspartate-histidine-serine, wherein the relative order of these amino acids reads from the amino to carboxy-terminus. More preferably at least one protease is a subtilisin related protease.

In one embodiment of the present invention, component (c) comprises at least one protease selected from the following: subtilisin from Bacillus amyloliquefaciens BPN' (described by Vasantha et al. (1984) J. Bacteriol. Volume 159, p. 811-819 and JA Wells et al. (1983) in Nucleic Acids Research, Volume 11, p. 7911-7925); subtilisin from Bacillus licheniformis (subtilisin Carlsberg; disclosed in EL Smith et al. (1968) in J. Biol Chem, Volume 243, pp. 2184-2191, and Jacobs et al. (1985) in Nucl. Acids Res, Vol 13, p. 8913-8926); subtilisin PB92 (original sequence of the alkaline protease PB92 is described in EP 283075 A2); subtilisin 147 and/or 309 (Esperase®, Savinase®, respectively) as disclosed in WO 89/06279; subtilisin from Bacillus lentus as disclosed in WO 91/02792, such as from Bacillus lentus DSM 5483 or the variants of Bacillus lentus DSM 5483 as described in WO 95/23221; subtilisin from Bacillus alcalophilus (DSM 11233) disclosed in DE 10064983; subtilisin from Bacillus gibsonii (DSM 14391) as disclosed in WO 2003/054184; subtilisin from Bacillus sp. (DSM 14390) disclosed in WO 2003/056017; subtilisin from Bacillus sp. (DSM 14392) disclosed in WO 2003/055974; subtilisin from Bacillus gibsonii (DSM 14393) disclosed in WO 2003/054184; subtilisin having SEQ ID NO: 4 as described in WO 2005/063974; subtilisin having SEQ ID NO: 4 as described in WO 2005/103244; subtilisin having SEQ ID NO: 7 as described in WO 2005/103244; and subtilisin having SEQ ID NO: 2 as described in application DE 102005028295.4.

In one embodiment, at least one subtilisin is subtilisin 309 (which might be called Savinase herein) as disclosed as sequence a) in Table I of WO 89/06279 or a variant thereof which is at least 80% similar and/or identical thereto and has proteolytic activity.

Examples of useful proteases in accordance with the present invention comprise the variants of subtilisin protease derived from SEQ ID NO:22 as described in EP 1921147 (which is the sequence of mature alkaline protease from Bacillus lentus DSM 5483) with amino acid substitutions in one or more of the following positions: 3, 4, 9, 15, 24, 27, 33, 36, 57, 68, 76, 77, 87, 95, 96, 97, 98, 99, 100, 101, 102, 103, 104, 106, 118, 120, 123, 128, 129, 130, 131, 154, 160, 167, 170, 194, 195, 199, 205, 206, 217, 218, 222, 224, 232, 235, 236, 245, 248, 252 and 274 (according to the BPN' numbering), which have proteolytic activity. In one embodiment, such a protease is not mutated at positions Asp32, His64 and Ser221 (according to BPN' numbering).

Component (c) preferably comprises at least one protease variant having proteolytic activity which is at least 85%, at least 90%, at least 91%, at least 92%, at least 93%, at least 94%, at least 95%, at least 96%, at least 97%, at least 98% or at least 99% identical when compared to the full-length polypeptide sequence of the parent enzyme as disclosed above.

In one embodiment, at least one protease comprised in component (b) has SEQ ID NO:22 as described in EP 1921147, or a protease which is at least 80% identical thereto and has proteolytic activity. In one embodiment, said protease is characterized by having at least amino acid glutamic acid (E), or aspartic acid (D), or asparagine (N), or glutamine (Q), or alanine (A), or glycine (G), or serine (S) at position 101 (according to BPN' numbering) and has proteolytic activity. In one embodiment, said protease comprises one or more further substitutions: (a) threonine at position 3 (3T), (b) isoleucine at position 4 (41), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or 217G), (i) combinations of two or more amino acids according to (a) to (h).

In one embodiment, at least one protease is at least 80% identical to SEQ ID NO:22 as described in EP 1921147 and is characterized by comprising one amino acid (according to (a)-(h)) or combinations according to (i) together with the amino acid 101E, 101D, 101N, 101Q, 101A, 101G, or 101S (according to BPN' numbering) and having proteolytic activity. In one embodiment, said protease is characterized by comprising the mutation (according to BPN' numbering) R101E, or S3T + V4I + V205I, or R101E and S3T, V4I, and V205I, or S3T + V4I + V199M + V205I + L217D, and having proteolytic activity.

In one embodiment, protease according to SEQ ID NO:22 as described in EP 1921147 is characterized by comprising the mutation (according to BPN' numbering) S3T + V4I + S9R + A15T + V68A + D99S + R101S + A103S + I104V + N218D, and having proteolytic activity.

In one embodiment, at least one protease is a protease 80% identical to SEQ ID NO:22 as described in EP 1921147 having R101E.

In one embodiment, detergent formulations comprise a combination of at least two proteases, preferably selected from the group of serine endopeptidases (EC 3.4.21), more preferably selected from the group of subtilisin type proteases (EC 3.4.21.62) - all as disclosed above.

Proteases herein are active proteins exerting "protease activity" or "proteolytic activity". Proteolytic activity is related to the rate of degradation of protein by a protease or proteolytic enzyme in a defined course of time. The methods for analyzing proteolytic activity are well-known in the literature (see e.g. Gupta et al. (2002), Appl. Microbiol. Biotechnol. 60: 381-395). Herein, proteolytic activity is determined by using Succinyl-Ala-Ala-Pro-Phe-p-nitroanilide (Suc-AAPF-pNA, short AAPF; see e.g. DelMar et al. (1979), Analytical Biochem 99, 316-320) as substrate. pNA is cleaved from the substrate molecule by proteolytic cleavage, resulting in release of yellow color of free pNA which can be quantified by measuring $OD_{405}$.

Proteolytic activity usually is provided in units per gram enzyme. For example, 1 U protease regularly is defined as the amount of protease which sets free 1 μmol folin-positive amino acids and peptides (as tyrosine) per minute at pH 8.0 and 37°C (casein as substrate).

In one embodiment, detergent formulations of the invention comprise at least one lipase.

"Lipases", "lipolytic enzyme", "lipid esterase", all refer to an enzyme of EC class 3.1.1 ("carboxylic ester hydrolase"). Lipase means active protein having lipase activity (or lipolytic activity; triacylglycerol lipase, EC 3.1.1.3), cutinase activity (EC 3.1.1.74; enzymes having cutinase activity may be called cutinase herein), sterol esterase activity (EC 3.1.1.13) and/or wax-ester hydrolase activity (EC 3.1.1.50).

Preferably at least one lipase comprises a catalytic triad comprises the amino acids aspartate-histidine-serine, wherein the relative order of these amino acids reads from the amino to carboxy-terminus is.

The methods for determining lipolytic activity are well-known in the literature (see e.g. Gupta et al. (2003), Biotechnol. Appl. Biochem. 37, p. 63-71). E.g. the lipase activity may be measured by ester bond hydrolysis in the substrate para-nitrophenyl palmitate (pNP-Palmitate, C:16) and releases pNP which is yellow and can be detected at 405 nm.

In one embodiment, at least one lipase is selected from fungal triacylglycerol lipase (EC class 3.1.1.3). Fungal triacylglycerol lipase is selected from Thermomyces lanuginosa lipase. In one embodiment, Thermomyces lanuginosa lipase is selected from polypetides which are at least 90%, at least 91%, at least 92%, at least 93%, at least 94%, at least 95%, at least 96%, at least 97%, at least 98% or at least 99% identical when compared to the full-length polypeptide sequence of amino acids 1-269 of SEQ ID NO:2 of US 5869438.

Thermomyces lanuginosa lipase is at least 80% identical to SEQ ID NO:2 of US 5869438 characterized by having amino acid T231R and N233R. Said Thermomyces lanuginosa lipase preferably further comprise one or more of the following amino acid exchanges: Q4V, V60S, A150G, L227G, P256K.

In one aspect, detergent formulations comprise at least one protease as disclosed above and/or at least one lipase as disclosed above.

In one embodiment, detergent formulations of the invention comprise at least one amylase. "Amylases" according to the invention (alpha and/or beta) include those of bacterial or fungal origin (EC 3.2.1.1 and 3.2.1.2, respectively). Preferably, the liquid compositions of the invention comprise at least one alpha-amylase (EC 3.2.1.1). Chemically modified or protein engineered mutants are included.

Amylases comprised in the liquid compositions of the invention according to the invention have "amylolytic activity" or "amylase activity" involving (endo)hydrolysis of glucosidic linkages in polysaccharides. alpha-amylase activity may be determined by assays for measurement of alpha-amylase activity which are known to those skilled in the art. Examples for assays measuring alpha-amylase activity are:

alpha-amylase activity can be determined by a method employing the Ethyliden-4-nitrophenyl-alpha-D-maltoheptaosid (EPS). D-maltoheptaoside is a blocked oligosaccharide which can be cleaved by an endo-amylase. Following the cleavage, the alpha-glucosidase included in the kit to digest the substrate to liberate a free PNP molecule which has a yellow color and thus can be measured by visible spectophotometry at 405nm. The slope of the time dependent absorption-curve is directly proportional to the specific activity (activity per mg enzyme) of the alpha-amylase in question under the given set of conditions.

At least one amylase comprised in the liquid compositions of the invention is selected from the following:

- amylases from Bacillus licheniformis having SEQ ID NO:2 as described in WO 95/10603. Suitable variants are described in WO 95/10603 comprising one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and

444 which have amylolytic activity. Variants are described in WO 94/02597, WO 94/018314, WO 97/043424 and SEQ ID NO:4 of WO 99/019467.

- amylases from B. stearothermophilus having SEQ ID NO:6 as disclosed in WO 02/10355 or an amylase with optionally having a C-terminal truncation over the wildtype sequence. Suitable variants of SEQ ID NO:6 include those comprising a deletion in positions 181 and/or 182 and/or a substitution in position 193.
- amylases from Bacillus sp.707 having SEQ ID NO:6 as disclosed in WO 99/19467. Preferred variants of SEQ NO: 6 are those having a substitution, a deletion or an insertion in one or more of the following positions: R181, G182, H183, G184, N195, 1206, E212, E216 and K269.
- amylases from Bacillus halmapalus having SEQ ID NO:2 or SEQ ID NO:7 as described in WO 96/23872, also described herein as SP-722. Preferred variants are described in WO 97/3296, WO 99/194671 and WO 2013/001078.
- amylases from Bacillus sp. DSM 12649 having SEQ ID NO:4 as disclosed in WO 00/22103.
- amylases from Bacillus strain TS-23 having SEQ ID NO:2 as disclosed in WO 2009/061380.
- amylases from Cytophaga sp. having SEQ ID NO:1 as disclosed in WO 2013/184577.
- amylases from Bacillus megaterium DSM 90 having SEQ ID NO:1 as disclosed in WO 2010/104675.
- amylases from Bacillus sp. comprising amino acids 1 to 485 of SEQ ID NO:2 as described in WO 00/60060.
- amylases from Bacillus amyloliquefaciens or variants thereof, preferably selected from amylases according to SEQ ID NO: 3 as described in WO 2016/092009.
- amylases having SEQ ID NO:12 as described in WO 2006/002643 or amylase variants comprising the substitutions Y295F and M202LITV within said SEQ ID NO:12.
- amylases having SEQ ID NO:6 as described in WO 2011/098531 or amylase variants comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L,I or V] within said SEQ ID NO:6.
- amylases having SEQ ID NO:1 as described in WO 2013/001078 or amylase variants comprising an alteration at two or more (several) positions corresponding to positions G304, W140, W189, D134, E260, F262, W284, W347, W439, W469, G476, and G477 within said SEQ ID NO:1.
- amylases having SEQ ID NO:2 as described in WO 2013/001087 or amylase variants comprising a deletion of positions 181+182, or 182+183, or 183+184, within said SEQ ID NO:2, optionally comprising one or two or more modifications in any of positions corresponding to W140, W159, W167, Q169, W189, E194, N260, F262, W284, F289, G304, G305, R320, W347, W439, W469, G476 and G477 within said SEQ ID NO:2.
- amylases which are hybrid alpha-amylases from above mentioned amylases as for example as described in WO 2006/066594;
- hybrid amylases according to WO 2014/183920 with A and B domains having at least 90% similarity and/or identity to SEQ ID NO:2 of WO 2014/183920 and a C domain having at least 90% similarity and/or identity to SEQ ID NO:6 of WO 2014/183920, wherein the hybrid amylase has amylolytic activity; preferably the hybrid alpha-amylase is at least 95% similar and/or identical to SEQ ID NO: 23 of WO 2014/183920 and having amylolytic activity;
- hybrid amylase according to WO 2014/183921 with A and B domains having at least 75% similarity and/or identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% similarity and/or identity to SEQ ID NO: 6 of WO 2014/183921 , wherein the hybrid amylase has amylolytic activity; preferably, the hybrid alpha-amylase is at least 95% similar and/or identical to SEQ ID NO: 30 as disclosed in WO 2014/183921 and having amylolytic activity.

According to the present invention, detergent formulations of the invention, in one embodiment, comprise a combination of at least two amylases as disclosed above.

In one embodiment, detergent formulations of the invention comprise at least one cellulase. "Cellulases", "cellulase enzymes" or "cellulolytic enzymes" are enzymes involved in hydrolysis of cellulose. At least one cellulase is selected from cellobiohydrolase (1,4-P-D-glucan cellobiohydrolase, EC 3.2.1.91), endo-ss-1,4-glucanase (endo-1,4-P-D-glucan 4-glucanohydrolase, EC 3.2.1.4) and ss-glucosidase (EC 3.2.1.21). Preferably, the liquid composition comprises at least one cellulase of the glycosyl hydrolase family 7 (GH7, pfam00840), preferably selected from endoglucanases (EC 3.2.1.4).

Assays for measurement of "cellulase activity" or "cellulolytic activity" are known to those skilled in the art. For example, cellulolytic activity may be determined by virtue of the fact that cellulase hydrolyses carboxymethyl cellulose to reducing carbohydrates, the reducing ability of which is determined colorimetrically by means of the ferricyanide reaction, according to Hoffman, W. S., J. Biol. Chem. 120, 51 (1937).

In one embodiment, detergent formulations of the invention comprise at least one cellulase selected of the glycosyl hydrolase family 7 (GH7, pfam00840), preferably selected from endoglucanases (EC 3.2.1.4).

In one embodiment, at least one cellulase is selected from cellulases comprising a cellulose binding domain.

In one embodiment, at least one cellulase is selected from cellulases comprising a catalytic domain only, meaning that the cellulase lacks cellulose binding domain.

In one embodiment, detergent formulations of the invention comprise at least one cellulase originating from Humicola insolens DSM 1800, Bacillus sp, Thielavia terrestris, Fusarium oxysporum, Sordaria fimicola and Trichoderma reesei.

In one embodiment, detergent formulations of the invention comprise at least one Humicola insolens DSM 1800 endoglucanase (EC 3.2.1.4) having the amino acid sequence as disclosed in position 21-435 of SEQ ID NO:2 of WO 2018/224544, and variants which are preferably at least 95% identical thereto.

In one embodiment, detergent formulations of the invention comprise at least a Bacillus sp. cellulase (EC 3.2.1.4) selected from a polypeptide which is at least 80% similar and/or identical to the amino acid sequence of position 1 to position 773 of SEQ ID NO: 2 of WO 2004/053039 or a catalytically active fragment thereof. In one embodiment, at least one cellulase is a mature polypeptide which is at least 95% identical to an amino acid sequence according to SEQ ID NO:1 of WO 2018/224544.

In one embodiment, detergent formulations of the invention comprise at least a Thielavia terrestris cellulase (EC 3.2.1.4) having a polypeptide at least 80% similar and/or identical to the amino acid sequence of position 1 to position 299 of SEQ ID NO: 4 of WO 2004/053039 or a catalytically active fragment thereof. In one embodiment, at least one cellulase is a mature polypeptide which is at least 95% identical to an amino acid sequence according to SEQ ID NO:4 of WO 2018/224544.

In one embodiment, at least one cellulase is a mature Sordaria fimicola cellulase which is at least 95% identical to an amino acid sequence according to SEQ ID NO:5 of WO 2018/224544.

According to the present invention, the liquid compositions of the invention, in one embodiment, comprise a combination of at least two cellulases, preferably selected from endoglucanases (EC 3.2.1.4) as disclosed above.

In one embodiment, detergent formulations of the invention comprise at least one mannanase. "Mannanases" according to the invention are mannan degrading enzymes selected from β-mannosidase (EC 3.2.1.25), endo-1,4-β-mannosidase (EC 3.2.1.78), and 1,4-β-mannobiosidase (EC 3.2.1.100). Preferably, at least one mannan degrading enzyme is selected from the group of endo-1,4-β-mannosidase (EC 3.2.1.78), a group of enzymes which may be called endo-β-1,4-D-mannanase, β-mannanase, or mannanase herein.

Mannan degrading activity may be determined according to standard test procedures known in the art, such as using carob galactomannan dyed with Remazol Brilliant Blue as described in McCleary, B. V. (1978). Carbohydrate Research, 67(1), 213-221. Another method for testing mannan degrading activity uses detection of reducing sugars when incubated with substrate such as guar gum or locust bean gut - for reference see Miller, G. L. Use of Dinitrosalicylic Acid Reagent for Determination of Reducing Sugars. Analytical Chemistry 1959; 31: 426-428.

In one embodiment, detergent formulations of the invention comprise at least one mannanase selected from alkaline mannanase of Family 5 or 26. The term "alkaline mannanase" is meant to encompass mannanases having an enzymatic activity at a given pH ranging from 7 to 12, preferably 7.5 to 10.5.

At least one mannanase comprised in detergent formulations of the invention may be selected from GH5 family mannanase. In one embodiment, at least one mannanase originates from Bacillus clausii (Man6, SEQ ID NO:12, WO 2018/184767) or variants at least 90% identical thereto. In one embodiment, at least one mannanase originates from Bacillus hemicellulosilyticus (Man7, SEQ ID NO:16 of WO 2018/184767) and variants thereof, e.g. as disclosed in WO 2018/220274. In one embodiment, at least one mannanase originates from Virgibacillus soli (Man14, SEQ ID NO:20 of WO 2018/184767) and variants at least 90% identical thereto. Preferably, at least one mannanase has a polypeptide sequence 95% identical to a polypeptide sequence of SEQ ID NO:20 of WO 2018/184767, preferably having at least one substitution selected from A101V, E405G, and Y459F.

In one embodiment, at least one mannanase comprised in detergent formulations of the invention is selected from WO 2009/074685 and variants at least 90% identical thereto.

In one embodiment, at least one mannanase comprised in detergent formulations of the invention is selected from mannanases originating from Trichoderma organisms, such as disclosed in WO 93/24622 and WO 2008/009673.

In one embodiment, at least one mannanase comprised in detergent formulations of the invention is selected from mannanases having a sequence according to positions 31-490 of SEQ ID NO:388 of WO 2005/003319 and variants which are preferably at least 90% identical thereto.

According to the present invention, detergent formulations of the invention, in one embodiment, comprise a combination of at least two mannanases, preferably one of them being an alkaline mannanase; at least one mannanase is selected from the group of endo-1,4-β-mannosidase (EC 3.2.1.78) as disclosed above.

In one embodiment, detergent formulations of the invention comprise at least one DNAse. DNAses catalyze the hydrolytic cleavage of phosphodiester linkages in DNA. The DNAses are classified e.g. in E.C. 3.1.11, E.C. 3.1.12, E.C. 3.1.15, E.C. 3.1.16, E.C. 3.1.21, E.C 3.1.22, E.C 3.1.23, E.C 3.1.24 and E.C.3.1.25 as well as EC 3.1.21.X, where X=1, 2, 3, 4, 5, 6, 7, 8 or 9.

DNAse activity can be determined by using the DNAseAlert™ Kit (11-02-01-04, IDT Intergrated DNA Technologies) according to the supplier's manual. Briefly, 95 μl DNase sample is mixed with 5 μl substrate in a microtiter plate, and fluorescence is immediately measured using e.g. a Clariostar microtiter reader from BMG Labtech (536 nm excitation, 556 nm emission).

In one embodiment, at least one DNAse comprised in detergent formulations of the invention is selected from polypeptides having an amino acid sequence which is at least 80% identical to SEQ ID NO: 1-24 and SEQ ID NO: 27-28 of WO 2019/081724 and WO 2019/081721. Variant DNAses comprises one or both of the motifs [D,M,L][S,T]GYSR[D,N] (SEQ ID NO: 25 of WO 2019/081724), and ASXNRSKG (SEQ ID NO: 26 of WO 2019/081724).

In one embodiment, at least one DNAse is selected from polypeptides comprising one or more of the motifs [G,Y,W,F,A,H]NI[R,Q,D,E,V] (SEQ ID NO: 73 of WO 2017/060493), SDH[D,H,L]P [SEQ ID NO: 74 of WO 2017/060493) and GGNI[R,Q] (SEQ ID NO: 75 of WO 2017/060493).

According to the present invention, the liquid compositions of the invention, in one embodiment, comprise a combination of at least two DNAses.

In one embodiment, detergent formulations of the invention comprise at least one surfactant. At least one surfactant may be introduced into the detergent formulation by using GPF as a detergent component. At least one surfactant may additionally added for comprising surfactant in effective amounts in detergent formulation.

Detergent formulations in one embodiment comprise at least one non-ionic surfactant (NIS) as disclosed herein.

Detergent formulations in one embodiment comprise at least one amphoteric surfactant (AS) as disclosed herein.

Detergent formulations in one embodiment comprise at least one anionic surfactant (AIS) of general formula (AIS-I):

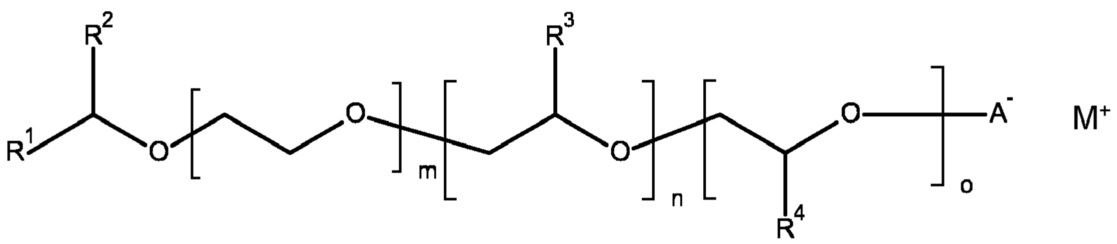

(AIS-I)

Wherein in formula (AIS-I) the following applies:

$R^1$ is selected from $C_1$-$C_{23}$-alkyl (such as 1-, 2-, 3-, 4- $C_1$-$C_{23}$-alkyl) and $C_2$-$C_{23}$-alkenyl, wherein alkyl and/or alkenyl are linear (straight-chain; n-) or branched, and wherein 2-, 3-, or 4-alkyl; examples are n-$C_7H_{15}$, n-$C_9H_{19}$, n-$C_{11}H_{23}$, n-$C_{13}H_{27}$, n-$C_{15}H_{31}$, n-$C_{17}H_{35}$, i-$C_9H_{19}$, i-$C_{12}H_{25}$.

$R^2$ is selected from H, $C_1$-$C_{20}$-alkyl and $C_2$-$C_{20}$-alkenyl, wherein alkyl and/or alkenyl are linear (straight-chain; n-) or branched.

$R^3$ and $R^4$, each independently selected from $C_1$-$C_{16}$-alkyl, wherein alkyl is linear (straight-chain; n-) or branched; examples are methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl, isodecyl.

$A^-$ is selected from $-RCOO^-$, $-SO_3^-$ and $RSO_3^-$, wherein R is selected from linear (straight-chain; n-) or branched $C_1$-$C_8$-alkyl, and $C_1$-$C_4$ hydroxyalkyl, wherein alkyl is. Compounds might be called (fatty) alcohol/alkyl (ethoxy/ether) sulfates [(F)A(E)S] when $A^-$ is $SO_3^-$, (fatty) alcohol/alkyl (ethoxy/ether) carboxylat [(F)A(E)C] when $A^-$ is $-RCOO^-$.

$M^+$ is selected from H and salt forming cations. Salt forming cations usually are monovalent or multivalent; hence $M^+$ equals 1/v $M^{v+}$. Examples include but are not limited to sodium, potassium, magnesium, calcium, ammonium, and the ammonium salt of mono-, di, and triethanolamine.

The integers of the general formula (AIS-I) are defined as follows:
m is in the range of zero to 200, preferably 1-80, more preferably 3-20; n and o, each independently in the range of zero to 100; n preferably is in the range of 1 to 10, more preferably 1 to 6; o preferably is in the range of 1 to 50, more preferably 4 to 25. The sum of m, n and o is at least one, preferably the sum of m, n and o is in the range of 5 to 100, more preferably in the range of from 9 to 50.

Anionic surfactants of the general formula (AIS-I) can be of any structure, block copolymers or random copolymers.

In a preferred embodiment, the detergent formulation of the invention comprises at least one anionic surfactant according to formula (AIS-I), wherein $R^1$ is n-$C_{11}H_{23}$, $R^2$ is H, $A^-$ is $SO_3^-$, m, n and o being 0. $M^+$ preferably is $NH_4^+$. Such compounds may be called ammonium lauryl sulfate (ALS) herein.

In a preferred embodiment, the detergent formulation of the invention comprises at least one anionic surfactant according to formula (AIS-I), wherein $R^1$ is n-$C_{11}H_{23}$, $R^2$ is selected from H, $A^-$ is $SO_3^-$, m being 2-5, preferably 3, and n and o being 0. $M^+$ preferably is $Na^+$. Such compounds may be called laurylethersulfates (LES) herein, preferably sodium laurylethersulfates (SLES).

The detergent formulation, in one embodiment, comprises at least two anionic surfactants, selected from compounds of general formula (AIS-I), wherein one of said anionic surfactants is characterized in $R^1$ being $C_{11}$, $R^2$ being H, m being 2, n and o = 0, $A^-$ being $SO_3^-$, $M^+$ being $Na^+$ and the other surfactant is characterized in $R^1$ being $C_{13}$, $R^2$ being H, m being 2, n and o = 0, $A^-$ being $SO_3^-$, $M^+$ being $Na^+$.

Typically, laundry detergent formulations comprise compounds according to formula AIS-I.

In one embodiment, the detergent formulation comprises at least one anionic surfactant selected from compounds of general formula (AIS-II):

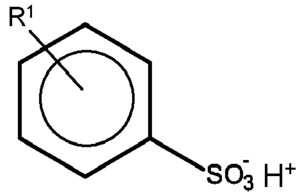

(AIS-II)

wherein $R^1$ in formula (AIS-II) is $C_{10}$-$C_{13}$ alkyl. Detergent formulations of the invention, in one embodiment, comprise salts of compounds according to formula (AIS-II), preferably sodium salts.

In a preferred embodiment, the detergent formulation of the invention comprise at least two anionic surfactants, selected from compounds of general formula (AIS-II), wherein one of said anionic surfactants is characterized in $R^1$ being $C_{10}$, and the other surfactant is characterized in $R^1$ being $C_{13}$. Compounds like this may be called LAS (linear alkylbenzene sulfonates) herein.

Depending on whether the detergent of the invention is determined for laundry purposes, automatic dishwashing purposes or manual hard surface cleaning purposes, the detergent formulation comprises a different set of surfactants in amounts applicable for the purpose.

In one embodiment, detergent formulations comprising the components of GPM of the invention are liquid laundry detergents. Usually, laundry detergents comprise relatively high amounts of surfactants, preferably selected from at least one anionic surfactant according to formula (AIS-I) and/or at least one anionic surfactant according to formula (AIS-II) and/or at least one non-ionic surfactant according to formula (NIS-IVa).

In one embodiment, liquid laundry detergents of the invention, comprise at least one hydrolase as disclosed herein. Preferably, at least one protease as disclosed herein is combined with one or more detergent components by addition of the liquid composition of the invention. Preferably, at least one protease is comprised in amounts of about 0.005% to 0.15% by weight, more preferably about 0.01% to 0.1% by weight, all relative to the total weight of the detergent formulation.

In one embodiment, liquid laundry detergents additionally comprise at least one lipase as disclosed herein, preferably in amounts of about 0.001% to 0.005%, more preferably 0.001% to 0.002% by weight, all relative to the total weight of the detergent formulation. AT least one lipase is selected from fungal triacylglycerol lipase is selected from Thermomyces lanuginosa lipase and variants thereof as disclosed herein.

In one embodiment, liquid laundry detergents additionally comprise at least one alpha-amylase as disclosed herein, preferably in amounts of about 0.001% to 0.015%, more preferably 0.002% to 0.015% by weight, all relative to the total weight of the detergent formulation.

In one embodiment, liquid laundry detergents additionally comprise at least one cellulase as disclosed herein, preferably in amounts of about 0.001% to 0.01%, more preferably 0.002% to 0.009% by weight, all relative to the total weight of the detergent formulation. At least one cellulase is selected from endoglucanases (EC 3.2.1.4), preferably those having the amino acid sequence disclosed in Fig. 14A-E of WO 91/17244 and variants thereof as disclosed herein.

In one embodiment, liquid laundry detergents additionally comprise at least one mannanase as disclosed herein, preferably in amounts of about 0.0005% to 0.005%, more preferably 0.0005% to 0.002% by weight, all relative to the total weight of the detergent formulation. At least one mannanase is selected from endo-1,4-β-mannosidase (EC 3.2.1.78) as disclosed herein.

In one embodiment, liquid laundry detergents comprise at least one aminocarboxylate selected from ethylenediaminetetraacetic acid (EDTA), diethylenetriaminepentaacetic acid (DTPA), methylglycine diacetate (MGDA), and glutamic acid diacetate (GLDA), all as disclosed above in amounts in the range of 1% to 10% by weight, in the range of 1.5% to 7% by weight, or in the range of 2% to 5% by weight all relative to the total weight of the liquid laundry detergent.

In one embodiment, liquid laundry detergents comprise citric acid in amounts in the range of 0.1% to 10% by weight, in the range of 0.5% to 8% by weight, in the range of 1% to 5% by weight, or in the range of 2% to 4% by weight, all relative to the total weight of the detergent formulation; the citric acid in one embodiment is provided as a mixture with formiate, e.g. Na-citrate: Na-formiate=9:1.

In one embodiment, liquid laundry detergents comprise at least one phosphonate as disclosed herein, preferably selected from HEDP and DTPMP, in amounts in the range of 0.5% to 3.0% by weight, or in the range of 1.0% to 2.5% by weight, all relative to the total weight of the detergent formulation.

In one embodiment, liquid laundry detergents comprise at least one ethoxylated polyethylenimine polymer which is based on a polyethylene core and a polyethylene oxide shell. Preferably, polyethylene imine core molecules are polyethylene imines with average molecular weight Mw in the range of 500 to 5000 g/mol. More preferred is a molecular weight from 500 to 1000 g/mol, even more preferred is an Mw of 600-800 g/mol. The ethoxylated polymer has in average 5 to 50, preferably 10 to 30 and even more preferably 15 to 25 EO (ethoxylate) groups per-NH group, resulting in an average molecular weight Mw in the range from 5,000 to 20,0000, preferably 8,000 to 100,000, more preferably 8,000 to 50,000, even more preferably 10,000 to 30,000, and most preferably 10,000 to 20,000) g/mol. Preferably at least one ethoxylated polyethyleneimine polymer is comprised in amounts ranging from about 0.5% to 5% by weight, from about 1% to 4%, or from about 1.5% to 3% by weight, all relative to the total weight of the liquid laundry detergent.

In one embodiment, liquid laundry detergents comprise at least one ethoxylated hexamethylene diamine polymer, which is preferably quaternized and - optionally but preferably- sulfated, preferably with an average molecular weight Mw in the range from 2000 to 10,000 g/mol, more preferably 3,000-8,000, most preferably 4,000-6,000. Preferably, ethoxylated hexamethylene diamine polymer, which is quaternized and- optionally but preferably- sulfated, contains in average 10 to 50, preferably 15 to 40 and even more preferably 20 to 30 EO (ethoxylate) groups per-NH group, resulting in an average molecular weight $M_W$ in the range from 2,000 to 10,000 g/mol, more preferably 3,000-8,000, most preferably 4,000-6,000. In a preferred embodiment the ethoxylated hexamethylene diamine is quaternized and also sulfated, preferably bearing 2 cationic ammonium groups and 2 anionic sulfate groups. Preferably at least one ethoxylated hexamethylene diamine polymer is comprised in amounts ranging from about 0.5% to 5% by weight, from about 1% to 4%, or from about 1.5% to 3% by weight, all relative to the total weight of the liquid laundry detergent.

In one embodiment, detergent formulations comprising the components of GPM of the invention are liquid manual hard surface detergents, preferably manual dishwashing detergents.

Liquid manual hard surface detergents preferably comprises at least one surfactant selected from ethoxylated or propoxylated sorbitan esters, amine oxides or alkyl polyglycosides, especially linear $C_4$-$C_{18}$-alkyl polyglucosides and branched $C_8$-$C_{18}$-alkyl polyglycosides such as compounds of general average formula (APG) are likewise suitable. APGs based on Guerbet alcohols are also suitable.

In one embodiment, liquid hard surface detergents comprise at least one amphoteric surfactant according to formula (AS-IIc), wherein preferably $R^{11}$ is $C_{11}$, r=3, $R^{12}$ and $R^{13}$ are $C_1$ alkyl, and $A^-$ is carboxyl. Preferably, such an amphoteric surfactant is comprised in liquid manual dishwashing detergents in amounts ranging from 1% to 10% by weight, 2% to 8% by weight, or 3% to 6% by weight, all relative to the total weight of the liquid manual hard surface detergent.

In one embodiment, liquid manual hard surface detergents comprise at least one amphoteric surfactant according to formula (AS-IV), wherein preferably $R^{18}$ is $C_1$ alkyl, x is 0 and $R^{16}$ is $C_{12}$. Preferably, such an amphoteric surfactant is comprised in liquid manual hard surface detergents in amounts ranging from 0.5% to 5% by weight, 1% to 4% by weight, or 1.5% to 3% by weight, all relative to the total weight of the liquid manual hard surface detergent.

In one embodiment, liquid manual hard surface detergents comprise at least two anionic surfactants, selected from compounds of general formula (AIS-I), wherein one of said anionic surfactants is characterized in $R^1$ being $C_{11}$, $R^2$ being H, m being 2, n and o = 0, A being $SO_3^-$, $M^+$ being $Na^+$ and the other surfactant is characterized in $R^1$ being $C_{13}$, $R^2$ being H, m being 2, n and o = 0, $A^-$ being $SO_3^-$, $M^+$ being $Na^+$. Preferably, such a mixture of anionic surfactants is comprised in liquid manual hard surface detergents in amounts ranging from 4% to 12% by weight, 5% to 11% by weight, or 6% to 10% by weight, all relative to the total weight of the liquid manual hard surface detergent.

In one embodiment, liquid manual hard surface detergents of the invention, comprise at least one hydrolase as disclosed herein. Preferably, at least one protease is comprised in amounts of about 0.002% to 0.25% by weight, more preferably about 0.005% to 0.2% by weight, all relative to the total weight of the manual hard surface detergent.

In one embodiment, liquid manual hard surface detergents additionally comprise at least one alpha-amylase as disclosed herein, preferably in amounts of about 0.001% to 0.005%, more preferably 0.001 to 0.004% by weight, all relative to the total weight of the manual hard surface detergent.

In one embodiment, liquid manual hard surface detergents comprise at least one ethoxylated polyethylenimine polymer which is based on a polyethylene core and a polyethylene oxide shell. Preferably, polyethylene imine core molecules are polyethylene imines with average molecular weight Mw in the range of 500 to 5000 g/mol. More preferred is a molecular weight from 500 to 1000 g/mol, even more preferred is an Mw of 600-800 g/mol. The ethoxylated polymer has in average 5 to 50, preferably 10 to 30 and even more preferably 15 to 25 EO (ethoxylate) groups per-NH group, resulting in an average molecular weight Mw in the range from 5,000 to 20,0000, preferably 8,000 to 100,000, more preferably 8,000 to 50,000, even more preferably 10,000 to 30,000, and most preferably 10,000 to 20,000) g/mol. Preferably at least one ethoxylated polyethyleneimine polymer is comprised in amounts ranging from about 0.1% to 3% by weight, from about 0.5% to 2.5%, or from about 1% to 2% by weight, all relative to the total weight of the liquid manual hard surface detergent.

In one embodiment, detergent formulations comprising the components of GPM of the invention are liquid automated dishwashing detergents.

Usually, automated dishwashing detergents do not comprise anionic surfactants. Preferably, liquid automated dishwashing detergents of the invention comprise at least one non-ionic surfactant according to formula (NIS-I), more preferably wherein $R^1$ is n-$C_8$ alkyl, $R^2$ is branched $C_{11}$ alkyl, AO is $CH_2$-$CH_2$-O, and x is 22. The automated dishwashing detergents preferably comprise such compounds in amounts in the range of about 0.3% to 10% by weight, in the range of about 0.5% to 5% by weight, or in the range of about 1% to 3%, all relative to the total weight of the liquid automated dishwashing detergent.

In one embodiment, liquid automated dishwashing detergents comprise at least one aminocarboxylate as disclosed above in amounts of 5% to 15% by weight relative to the total weight of the detergent formulation.

Preferably, automated dishwashing detergents comprises a builder system comprising

- ethylenediaminetetraacetic acid (EDTA) and/or diethylenetriaminepentaacetic acid (DTPA) and/or methylglycine diacetate (MGDA) and/or glutamic acid diacetate (GLDA), as disclosed above in amounts in the range of 0.1% to 15% by weight, in the range of 1% to 10% by weight, in the range of 3% to 8% by weight, or in the range of 2.5% to 5% by weight all relative to the total weight of the detergent formulation;

- optionally citric acid in amounts in the range of 0.1% to 10% by weight, in the range of 0.5% to 8% by weight, in the range of 1% to 5% by weight, or in the range of 2% to 4% by weight, all relative to the total weight of the detergent formulation; the citric acid in one embodiment is provided as a mixture with formiate, e.g. Na-citrate:Na-formiate=9:1;

- optionally at least one phosphonate, preferably selected from derivatives polyphosphonic acids such as of diphosphonic acid such as sodium salt of HEDP, and derivatives of aminopolyphosphonic acid such as aminoalkylene phosphonic acids such as DTPMP in amounts in the range of 0.1% to 5% by weight, in the range of 0.5% to 3% by weight, or in the range of 1% to 2% by weight, all relative to the total weight of the detergent formulation;

- optionally at least one polycarboxylate selected from homopolymers with the repeating monomer being the same unsaturated carboxylic acid, such as polyacrylic acid (PAA) and copolymers with the repeating monomers being at least two different unsaturated carboxylic acids, such as copolymers of acrylic acid with methacrylic acid, copolymers of acrylic acid or methacrylic acid and maleic acid and/or fumaric acid, in amounts in the range of 0% to 10% by weight, 0.5% to 7% by weight, 1% to 5% by weight, or 2.5% to 5% by weight, all relative to the total weight of the detergent formulation; said homopolymers of poly acrylic acid may be partially neutralized or sulfonated.

In one embodiment, liquid automated dishwashing detergents of the invention, comprise at least one hydrolase as disclosed herein. At least one protease as disclosed herein is combined with one or more detergent components by addition of the liquid composition of the invention. Preferably, at least one protease is comprised in amounts of about 0.10% to 0.25% by weight, more preferably about 0.12% to 0.21% by weight, all relative to the total weight of the detergent formulation.

In one embodiment, liquid automated dishwashing detergents additionally comprise at least one alpha-amylase as disclosed herein, preferably in amounts of about 0.002% to 0.015%, more preferably 0.004 to 0.01% by weight, all relative to the total weight of the detergent formulation.

In one embodiment liquid automatic dishwashing detergents comprise at least one zinc salt. Zinc salts are preferably selected from water-soluble and water-insoluble zinc salts. In this connection, within the context of the present invention, water-insoluble is used to refer to those zinc salts which, in distilled water at 25°C, have a solubility of 0.1 g/l or less. Zinc salts which have a higher solubility in water are accordingly referred to within the context of the present invention as water-soluble zinc salts.

The zinc salt may be selected from zinc benzoate, zinc gluconate, zinc lactate, zinc formate, $ZnCl_2$, $ZnSO_4$, zinc acetate, zinc citrate, $Zn(NO_3)_2$, $Zn(CH_3SO_3)_2$ and zinc gallate, preferably $ZnCl_2$, $ZnSO_4$, zinc acetate, zinc citrate,

$Zn(NO_3)_2$, $Zn(CH_3SO_3)_2$ and zinc gallate.

In another embodiment of the present invention, zinc salt is selected from ZnO, ZnO·aq, $Zn(OH)_2$ and $ZnCO_3$. Preference is given to ZnO·aq.

In one embodiment of the present invention, zinc salt is selected from zinc oxides with an aver-age particle diameter (weight-average) in the range from 10 nm to 100 μm.

The cation in zinc salt can be present in complexed form, for example complexed with ammonia ligands or water ligands, and in particular be present in hydrated form. To simplify the notation, within the context of the present invention, ligands are generally omitted if they are water lig-ands.

Depending on how the pH of mixture according to the invention is adjusted, zinc salt can change. Thus, it is for example possible to use zinc acetate or $ZnCl_2$ for preparing formulation according to the invention, but this converts at a pH of 8 or 9 in an aqueous environment to ZnO, $Zn(OH)_2$ or ZnO·aq, which can be present in non-complexed or in complexed form.

Zinc salt preferably is present in liquid detergent formulations in dissolved or in colloidal form.

In one embodiment of the present invention, inventive automatic dishwashing formulations comprise in total in the range from 0.05 to 0.4% by weight of zinc salt, based in each case on the solids content of the formulation in question. Herein, the fraction of zinc salt is given as zinc or zinc ions. From this, it is possible to calculate the counterion fraction.

The invention in one aspect related to the method of preparing the detergent formulation of the invention by mixing in one or more steps the GPM or GPF of the invention with one or more detergent components in any order.

Enzyme stabilization in detergent formulation

In one aspect of the invention, GPM and/or GPF of the invention are used for stabilizing at least one hydrolase within a liquid detergent formulation.

At least one hydrolase comprised in the detergent formulation of the invention is storage stable within a liquid detergent formulation of the invention. In a preferred embodiment, the detergent formulation is a formulation selected from laundry detergent, automated dishwashing detergent and manual dishwashing detergent. The specific features of said detergent formulations are as disclosed above.

Storage stability relating to hydrolase activity within a detergent formulation, preferably means that the residual hydrolytic activity after storage for up to 28 days, up to 42 days, or up to 56 days at elevated temperatures of about 30°C or 37°C is at least 60%, at least 65%, at least 70%, at least 75%, at least 80%, or at least 85% when compared to the initial hydrolytic activity available before storage.

In one embodiment, at least one hydrolase comprised in the liquid detergent formulation of the invention has as a catalytic triad the amino acids aspartate, histidine and serine. Preferably, at least one hydrolase is a subtilisin proteases (EC 3.4.21.62), preferably a protease according to SEQ ID NO:22 as described in EP 1921147 or variants thereof having proteolytic activity, preferably a protease 80% identical to SEQ ID NO:22 as described in EP 1921147 having R101E.

In one embodiment of the invention, the in-detergent storage stability of at least one hydrolase is increased in the presence of at least one compound according to formula (TAC), preferably triethyl citrate, when compared to the in detergent-stability in liquid detergents lacking said compound. Preferably, at least one compound according to formula (TAC), preferably triethyl citrate is present in amounts of about 0.2% to 1% by weight, 0.3% to 0.9% by weight, 0.4% to 0.8% by weight, or 0.5% to 0.7% by weight, all relative to the total weight of the detergent formulation.

In one embodiment, the residual hydrolytic activity in in liquid detergent formulations of the invention comprising triethyl citrate after storage at elevated temperatures of about 30°C or 37°C for up to 42 days is at least 70%, at least 75%, at least 80%, at least 85%, or at least 90% when compared to the initial enzymatic activity available before storage. Increase of storage stability in the presence of triethyl citrate preferably means increase in residual enzymatic activity after storage by at least 30%, at least 25%, at least 20%, at least 15%, or at least 10% when compared to liquid detergent formulations lacking triethyl citrate.

Wash performance in detergent formulation

The invention relates to the use of liquid hydrolase-containing detergent formulations of the invention to remove stains and/or soils, preferably proteinaceous stains and/or starch-containing stains and/or cellulose-containing stains from surfaces to be cleaned such as textiles and hard surfaces.

In one embodiment, hydrolase-containing detergent formulations of the invention are used to increase wash performance towards proteinaceous stains and/or starch-containing stains and/or cellulose-containing stains after storage of the detergent formulation at 37°C for up to 42 days when compared to detergent formulations stored under the same storing condition but lacking GPM of the invention.

In one embodiment, detergent formulations of the invention are used to increase fat removal when compared

to the use of detergent formulations lacking GPM of the invention.

Wash performance relates to the ability to remove stains and/or soils under relevant cleaning conditions.

The term "laundering" relates to both household laundering and industrial laundering and means the process of treating textiles with a solution comprising a detergent formulation of the present invention. The laundering process usually is carried out by using technical devices such as a household or an industrial washing machine. Alternatively, the laundering process can be done by hand.

The term "textile" means any textile material including yarns (thread made of natural or synthetic fibers used for knitting or weaving), yarn intermediates, fibers, non-woven materials, natural materials, synthetic materials, as well as fabrics (a textile made by weaving, knitting or felting fibers) made of these materials such as garments (any article of clothing made of textile), cloths and other articles.

The term "fibers" includes natural fibers, synthetic fibers, and mixtures thereof. Examples of natural fibers are of plant (such as flax, jute and cotton) or animal origin, comprising proteins like collagen, keratin and fibroin (e.g. silk, sheeps wool, angora, mohair, cashmere). Examples for fibers of synthetic origin are polyurethane fibers such as Spandex® or Lycra®, polyester fibers, polyolefins such as elastofin, or polyamide fibers such as nylon. Fibers preferably include single fibers or parts of textiles such as knitwear, wovens, or nonwovens.

The term "hard surface cleaning" is defined herein as cleaning of hard surfaces wherein hard surfaces include any hard surfaces in the household, such as floors, furnishing, walls, sanitary ceramics, glass, metallic surfaces including cutlery or dishes. The term "hard surface cleaning" in one aspect means "dish washing" which refers to all forms of washing dishes, e.g. by hand or automatic dish wash (ADW). Dish washing includes, but is not limited to, the cleaning of all forms of crockery such as plates, cups, glasses, bowls, all forms of cutlery such as spoons, knives, forks and serving utensils as well as ceramics, plastics such as melamine, metals, china, glass and acrylics.

The present invention is directed to a process for cleaning laundry and/or hard surfaces, preferably dishware by contacting laundry and/or hard surfaces, preferably dishware with a detergent formulation of the invention as disclosed above.

The inventive washing and/or cleaning process is being carried out at temperatures in the range of from 10°C to 90°C. In embodiments wherein the inventive cleaning process is carried out as a laundering process, it is preferably carried out at a temperature in the range of from 10°C to 60°C, more preferably 20°C to 40°C. In embodiments wherein the inventive cleaning process is carried out as an automatic dishwashing process, it is preferably carried out at a temperature in the range of from 45°C to 65°C, more preferably 50°C to 60°C. Said temperatures refer to the temperature of the washing and/or cleaning water being used in the inventive process.

In one embodiment, the use of the detergent formulations of the invention increases the wash performance towards proteinaceous stains and/or starch-containing stains and/or cellulose-containing stains, when compared to detergent formulations lacking at least one protease as disclosed herein.

Preferably, the use of automated dishwashing detergents according to the invention increases the wash performance towards crème brûlée, more preferably when attached to porcelain surfaces, when compared to detergent formulations lacking at least one protease as disclosed herein. Preferably, when compared to detergent formulations lacking the components of the liquid composition of the invention.

## Examples

### I. graft polymer mixtures

General remarks:
As radical starter, tert.-butyl-peroctoate was used as a 25% by weight solution in tripropylene glycol ("solution of radical starter").

Comonomers for grafting:

Comonomers according to formula (CM-Ia): vinylactate (CM-Ia.1), vinylpropionate (CM-Ia.2), vinylbutyrate (n-butyric acid vinyl ester) (CM-Ia.3), vinyl 2-ethylhexanoate (CM-Ia.4), vinyllaurate (CM-Ia.5)

Comonomers according to formula (CM-Ic): 2-ethylhexylacrylate (CM-Ic.1), lauryl acrylate (CM-Ic.2)

Trialkyl citrate:

TAC.1: triethylcitrate
TAC.2: tripropylcitrate

Surfactant:

Surfactant (S.1): n-$C_8H_{17}$-CH(OH)-$CH_2$-O-$(EO)_{22}$-CH($CH_3$)-$CH_2$-O-n-$C_{10}H_{21}$
Surfactant (S.2): n-$C_{10}H_{21}$-CH(OH)-$CH_2$-O-$(EO)_{40}$-n-$C_{10}H_{21}$
Surfactant (S.3): n-$C_5H_{11}$CH(OH)-$CH_2$-O-$(EO)_{32}$-n-$C_{12}H_{25}$

Percentages refer to weight percent unless expressly noted otherwise.

Syntheses of inventive graft polymers (GP) and comparison copolymers

Example 1a:

A 4-l-vessel with stirrer and three feeds was charged with 1820 g polyethylene glycol ($M_n$: 4,000 g/mol) and heated to 90°C and purged with nitrogen. Solution of radical starter (125 g) was fed through feed 1 over a period of 7 hours. 15 minutes after the commencement of feed 1, an amount of 780 g vinylacetate (CM-la.1) was fed continuously through feed 2 within 6 hours. When feed 2 was completed, feed 3 (3 hours, continuous addition of 71.5 g solution of radical starter) commenced. When feed 3 had been completed the reaction mixture was stirred 100°C for another hour. Then, the pressure was set to 10 mbar, and volatile components were removed at 100°C and 10 mbar under stirring. The reaction mixture was then cooled to 85°C and 910g triethylcitrate (TAC.1) were added and stirred for 10 minutes. Inventive graft polymer mixture (GPM.1a) was obtained as a slightly yellowish liquid, 3,530 g.

Example 1b:

A 4-l-vessel with stirrer and three feeds was charged with 1820 g polyethylene glycol ($M_n$: 4,000 g/mol) and heated to 90°C and purged with nitrogen. Solution of radical starter (125 g) was fed through feed 1 over a period of 7 hours. 15 minutes after the commencement of feed 1, an amount of 780 g vinylacetate (CM-la.1) was fed continuously through feed 2 within 6 hours and. When feed 2 was 50% achieved (3h), feed 3 (3 hours) commenced. Through feed 3 71.5 g solution of radical starter and 910g triethylcitrate (TAC.1) were added continuously. When feed 3 had been completed the reaction mixture was stirred 100°C for another hour. Then, the pressure was set to 10 mbar, and volatile components were removed at 100°C and 10 mbar under stirring. The reaction mixture was then cooled to room temperature and the inventive graft polymer mixture (GPM.1b) was obtained as a slightly yellowish liquid, 3,540 g.

Example 1c:

1500 g of GPM.1a were heated to 60°C and 750 g of surfactant S.1 was added, a viscous liquid was obtained (GPF.1).

Example 2:

A 4-l-vessel with stirrer and three feeds was charged with 1940 g polyethylene glycol ($M_n$: 4,000 g/mol) and heated to 90°C and purged with nitrogen. Solution of radical starter (91 g) was fed through feed 1 over a period of 7 hours. 15 minutes after the commencement of feed 1, an amount of 825 g 2-ethylhexylacrylate (CM-Ic.1) was fed continuously through feed 2 within 6 hours. When feed 2 was completed, feed 3 (3 hours, 74.5 g solution of radical starter) commenced. When feed 3 had been completed the reaction mixture was stirred 100°C for another hour. Then, the pressure was set to 10 mbar, and volatile components were removed at 100°C and 10 mbar under stirring. Into the reaction mixture 760 g triethylcitrate (TAC.1) were added, and then cooled to ambient temperature under stirring. Inventive graft polymer mixture (GPM.2) was obtained as a off white liquid, 3530 g.

Example 3:

A 4-l-vessel with stirrer and three feeds was charged with 1927 g polyethylene glycol ($M_n$: 4,000 g/mol) and heated to 90°C and purged with nitrogen. Solution of radical starter (88 g) was fed through feed 1 over a period of 7 hours. 15 minutes after the commencement of feed 1, an amount of 831 g laurylacrylate (CM-Ic.2) was fed continuously through feed 2 within 6 hours. When feed 2 was completed, feed 3 (3 hours, 76.5 g of solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 840g triethylcitrate (TAC.1) were added, and then cooled to ambient temperature under stirring. Inventive graft polymer. Inventive graft polymer mixture (GPM.3) was obtained as a yellowish liquid, 3582 g.

Example 4:

A 4-l-vessel with stirrer and three feeds was charged with 2,353 g polyethylene glycol ($M_n$: 4,000 g/mol) and heated to 90°C and purged with nitrogen. Solution of radical starter (91 g) was fed through feed 1 over a period of 7 hours. 15 minutes after the commencement of feed 1, an amount of 483.5 g vinylproprionate (CM-la.2) was fed continuously through feed 2 within 6 hours. When feed 2 was finished, feed 3 (3 hours, 71.5 g of solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 760 g triethylcitrate (TAC.1) were added, and then cooled to ambient temperature under stirring. Inventive graft polymer was then cooled to ambient temperature. Inventive graft polymer mixture (GPM.4) was obtained as a clear liquid, 3775 g.

Example 5:

A 4-l-vessel with stirrer and three feeds was charged with 2,353 g polyethylene glycol ($M_n$: 4,000 g/mol) and heated to 90°C and purged with nitrogen. Solution of radical starter (46 g) was fed through feed 1 over a period of 7 hours. 15 minutes after the commencement of feed 1, an amount of 455 g vinylacetate (CM-la.1) was fed continuously through feed 2 within 5 hours. When feed 2 was completed, feed 3 (3 hours, 65 g of solution radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 930 g tripropylcitrate (TAC.2) were added, and then cooled to ambient temperature under stirring. Inventive graft polymer mixture (GPM.5) was obtained as a yellowish liquid, 3723 g.

Example 6:

A 4-l-vessel with stirrer and three feeds was charged with 1987 g polyethylene glycol ($M_n$: 4,000 g/mol) and heated to 90°C and purged with nitrogen. Solution of radical starter (91 g) was fed through feed 1 over a period of 7 hours. 15 minutes after the commencement of feed 1, an amount of 651 g vinylbutyrate (CM-la.3) was fed continuously through feed 2 within 6 hours. When feed 2 was completed, feed 3 (3 hours, 71.5 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 800 g triethylcitrate (TAC.1) were added, and then cooled to ambient temperature under stirring. Inventive graft polymer mixture (GPM.6) was obtained as a clear liquid, 3558 g.

Example 7:

A 4-l-vessel with stirrer and three feeds was charged with 2,270 g polyethylene glycol ($M_n$: 4,000 g/mol) and heated to 90°C and purged with nitrogen. Solution of radical starter (91 g) was fed through feed 1 over a period of 7 hours. 15 minutes after the commencement of feed 1, an amount of 567 g vinyl-2-ethylhexanoate (CM-la.4) was fed continuously through feed 2 within 6 hours. When feed 2 was completed, feed 3 (3 hours, 71.5 g of solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 560 g triethylcitrate (TAC.1) were added, and then cooled to ambient temperature under stirring. Inventive graft polymer mixture (GPM.7) was obtained as a clear liquid, 3520 g.

Example 8a:

A 4-l-vessel with stirrer and three feeds was charged with 2,270 g polyethylene glycol ($M_n$: 4,000 g/mol) and heated to 90°C and purged with nitrogen. Solution of radical starter (91 g) was fed through feed 1 over a period of 7 hours. 15 minutes after the commencement of feed 1, an amount of 367 g vinyl laurate (CM-la.5) was fed continuously through feed 2 within 6 hours. When feed 2 was completed, feed 3 (3 hours, 51.5 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 760 g triethylcitrate (TAC.1) were added, and then cooled to ambient temperature under stirring. Inventive graft polymer mixture (GPM.8) was obtained as a clear liquid, 3518 g.

Example 8b:

1500 g of GPM.8 were heated to 60°C and 750 g of surfactant S.3 was added, a viscous liquid was obtained (GPF.8).

Example 9a:

A 4-l-vessel with stirrer and three feeds was charged with 2,153 g polyethylene glycol ($M_n$: 8,000 g/mol) and heated to 90°C and purged with nitrogen. Solution of radical starter (46 g) was fed through feed 1 over a period of 7 hours. 15 minutes after the commencement of feed 1, an amount of 483.5 g vinyl laurate (CM-la.5) was fed continuously through feed 2 within 5 hours. When feed 2 was completed, feed 3 (3 hours, 46 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 860 g tripropylcitrate (TAC.2) were added, and then cooled to ambient temperature under stirring. Inventive graft polymer mixture (GPM.9) was obtained as a clear liquid, 3483 g.

Example 9b:

1500 g of GPM.9 were heated to 60°C and 500 g of surfactant S.1 was added, a viscous liquid was obtained (GPF.9).

Example 10a:

A 4-l-vessel with stirrer and three feeds was charged with 2,270 g of a di-block copolyether polyethylene/propylene glycol (weight ratio EO/PO: 1:1, $M_n$: 4,500 g/mol) and heated to 90°C and purged with nitrogen. Solution of radical starter (91 g) was fed through feed 1 over a period of 7 hours. 15 minutes after the commencement of feed 1, an amount of 567 g vinylacetate (CM-la.1) was fed continuously through feed 2 within 6 hours. When feed 2 was completed, feed 3 (3 hours, 71.5 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 760 g triethylcitrate (TAC.1) were added, and then cooled to ambient temperature under stirring. Inventive graft polymer mixture (GPM.10) was obtained as a clear liquid, 3718 g.

Example 10b:

1500 g of GPM.10 were heated to 60°C and 750 g of surfactant S.2 was added, a viscous liquid was obtained (GPF.10).

Example 11:

A 4-l-vessel with stirrer and three feeds was charged with 2,553 g of a di-block copolyether polyethylene/propylene glycol (weight ratio EO/PO: 2:3, $M_n$: 5,800 g/mol) and heated to 90°C and purged with nitrogen. Solution of radical starter (91 g) was fed through feed 1 over a period of 7 hours. 15 minutes after the commencement of feed 1, an amount of 283.5 g vinylpropionate (CM-la.2) was fed continuously through feed 2 within 6 hours. When feed 2 was completed, feed 3 (3 hours, 61.0 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 800 g triethylcitrate (TAC.1) were added, and then cooled to ambient temperature under stirring. Inventive graft polymer mixture (GPM.11) was obtained as a clear liquid, 3642 g.

Example 12a:

A 4-l-vessel with stirrer and three feeds was charged with 2,553 g of a di-block copolyether polyethylene/propylene glycol (weight ratio EO/PO: 1:1, $M_n$: 9,500 g/mol) and heated to 90°C and purged with nitrogen. Solution of radical starter (91 g) was fed through feed 1 over a period of 7 hours. 15 minutes after the commencement of feed 1, an amount of 283.5 g lauryl acrylate (CM-lc.2)was fed continuously through feed 2 within 6 hours. When feed 2 was completed, feed 3 (3 hours, 71.5 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 760 g triethylcitrate (TAC.1) were added, and then cooled to ambient temperature under stirring. Inventive graft polymer mixture (GPM.12) was obtained as an off-white liquid, 3713 g.

Example 12b:

1500 g of GPM.12 were heated to 60°C and 750 g of surfactant S.2 was added, a viscous liquid was obtained (GPF.12).

Example 13:

A 4-l-vessel with stirrer and three feeds was charged with 2,270 g of a di-block copolyether polyethylene/propylene glycol (weight ratio EO/PO: 2:3, $M_n$: 2,900 g/mol) and heated to 90°C and purged with nitrogen. Solution of radical starter (91 g) was fed through feed 1 over a period of 7 hours. 15 minutes after the commencement of feed 1, an amount of 467 g vinyl laurate (CM-la.5) was added continuously through feed 2 within 6 hours. When feed 2 was completed, feed 3 (3 hours, 61.5 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 860 g triethylcitrate (TAC.1) were added, and then cooled to ambient temperature under stirring. Inventive graft polymer mixture (GPM.13) was obtained as a clear liquid, 3587g.

Example 14:

A 4-l-vessel with stirrer and three feeds was charged with 2,270 g of polypropylene glycol ($M_n$: 2,000 g/mol) and heated to 90°C and purged with nitrogen. Solution of radical starter (91 g) was fed through feed 1 over a period of 7 hours. 15 minutes after the commencement of feed 1, an amount of 567 g vinylacetate (CM-la.1) was added continuously through feed 2 within 6 hours. When feed 2 was completed, feed 3 (3 hours, 71.5 g solution of radical starter) commenced. When feed 3 had been completed the reaction mixture was stirred 100°C for another hour. Then, the pressure was set to 10 mbar, and volatile components were removed at 100°C and 10 mbar under stirring. To the reaction mixture 560 g triethylcitrate (TAC.1) were added, and then cooled to ambient temperature under stirring. Inventive graft polymer mixture (GPM.14) was obtained as a viscous liquid, 3392 g.

Example 15:

A 4-l-vessel with stirrer and three feeds was charged with 1980 g polyethylene glycol ($M_n$: 1,500 g/mol) and heated to 90°C and purged with nitrogen. Solution of radical starter (125 g) was fed through feed 1 over a period of 7 hours. 15 minutes after the commencement of feed 1, an amount of 495 g vinylacetate (CM-la.1) was fed continuously through feed 2 within 6 hours. When feed 2 was completed, feed 3 (3 hours) commenced. Through feed 3 56 g solution of radical starter and 930 g triethylcitrate (TAC.1) were added continuously. When feed 3 had been completed the reaction mixture was stirred 125°C for 90 minutes. Then, the pressure was set to 40 mbar, and volatile components were removed at 125°C and 40 mbar under stirring. The reaction mixture was then cooled to room temperature and the inventive graft polymer mixture (GPM.15) was obtained as a yellowish liquid, 3,398 g.

Example 16:

A 4-l-vessel with stirrer and three feeds was charged with 1910 g polyethylene glycol (Mn: 4000 g/mol) and heated to 90°C and purged with nitrogen. Solution of radical starter (125 g) was fed through feed 1 over a period of 7 hours. 15 minutes after the commencement of feed 1, an amount of 70 g vinyllaurate (CM-la.5) was added within 5 minutes and also 430 g vinylacetate (CM-la.1) was fed continuously through feed 2 within 5 hours. After addition of feed 2 was completed, feed 3 (3 hours, 56 g solution of radical starter) commenced. Trough feed 3 910g triethylcitrate (TAC.1) were also added. After the addition of the radical starter had been completed the reaction mixture was stirred 125°C for 90 minutes. Then, the pressure was set to 40 mbar, and volatile components were removed at 125°C and 40 mbar under stirring. The reaction mixture was then cooled to room temperature and the inventive graft copolymer mixture (GPM.16) was obtained as a yellowish liquid, 3,382 g.

Control graft polymers were synthesized following the above protocols but with the following modifications.

Example 17:

The protocol of example 1a was followed, but no triethylcitrate was added, resulting in an off white solid (C-GP17).

Example 18

The protocol of example 4 was followed, but no triethylcitrate was added, resulting in an off white solid (C-GP18).

The properties of inventive graft polymer mixtures (GPM) and of comparative graft polymers (C-GP) are summarized in Table Ex-Ia.

Table Ex-Ia:

| | backbone | $M_n$ [g/mol] | comonomer | Wt. ratio graft base/ side chain | Wt. ratio TAC /GP |
|---|---|---|---|---|---|
| GPM. 1a | poly-EO | 4.000 | vinylacetate (CM-Ia.1) | 0.70 | 0.35; TAC.1 |
| GPM. 1b | poly-EO | 4.000 | vinylacetate (CM-Ia.1) | 0.70 | 0.35; TAC.1 |
| GPM.2 | poly-EO | 4.000 | 2-ethylhexylacrylate (CM-Ic.1) | 0.70 | 0.275; TAC.1 |
| GPM.3 | poly-EO | 4.000 | laurylacrylate (CM-Ic.2) | 0.70 | 0.30; TAC.1 |
| GPM.4 | poly-EO | 4.000 | vinyl propionate (CM-Ia.2) | 0.83 | 0.27; TAC.1 |
| GPM.5 | poly-EO | 4.000 | vinylacetate (CM-Ia.1) | 0.84 | 0.33; TAC.2 |
| GPM.6 | poly-EO | 4.000 | vinylbutyrate (CM-Ia.3) | 0.75 | 0.30; TAC.1 |
| GPM.7 | poly-EO | 4.000 | vinyl-2-ethylhexanoate (CM-Ia.4) | 0.80 | 0.20; TAC.1 |
| GPM.8 | poly-EO | 4.000 | vinyllaurate (CM-Ia.5) | 0.86 | 0.30; TAC.1 |
| GPM.9 | poly-EO | 8.000 | vinyllaurate (CM-Ia.5) | 0.82 | 0.33; TAC.2 |
| GPM. 10 | poly-EO-PO (1:1) | 4.500 | vinylacetate (CM-Ia.1) | 0.80 | 0.27; TAC.1 |
| GPM. 11 | poly-EO-PO (2:3) | 5.800 | vinyl propionate (CM-Ia.2) | 0.90 | 0.28; TAC.1 |
| GPM. 12 | poly-EO-PO (1:1) | 9.500 | vinylacetate (CM-Ia.1) | 0.84 | 0.31; TAC.1 |
| GPM. 13 | poly-EO-PO (2:3) | 2.900 | vinyllaurate (CM-Ia.5) | 0.83 | 0.31; TAC.1 |
| GPM. 14 | poly-PO | 2.000 | vinylacetate (CM-Ia.1) | 0.80 | 0.20; TAC.1 |
| GPM. 15 | poly-EO | 1.500 | vinylacetate (CM-Ia.1) | 0.80 | 0.38; TAC.1 |
| GPM. 16 | poly-EO | 4.000 | vinylacetate (CM-Ia.1) vinyllaurate (CM-Ia.5); in a weight ratio 6.1:1 | 0.80 | 0.38 |
| C-GP. 17 | poly-EO | 4.000 | vinylacetate (CM-Ia.1) | 0.70 | - |
| C-GP. 18 | poly-EO | 4.000 | vinyl propionate (CM-Ia.2) | 0.83 | - |

Table Ex-Ib: GPM + surfactant - GPF

| | Based on | GPM:surfactant |
|---|---|---|
| GPF.1 | GPM.1a | 2:1; S.1 |
| GPF.8 | GPM.8 | 2:1; S.3 |
| GPF.9 | GPM.9 | 3:1; S.1 |
| GPF.10 | GPM.10 | 2:1; S.2 |
| GPF.12 | GPM.12 | 2:1; S.1 |

II. laundry formulations

All laundry experiments were executed after a storage period of formulations (table Ex-IIb) of 6 weeks at 37°C. This pretreatment shall mimic storage of liquid detergent over 4 months at room temperature, which represents a typical, mean storage time from production until the end consumer uses the product and expects top fresh performance. This quality criteria are becoming more and more important as enzymes are used that can degrade in not appropriate formulations.

Base test formulations were manufactured by making base formulations L.I to L.IV by mixing the components according to Table Ex-IIa.

Table Ex-IIa: liquid laundry formulations

| Base formulation | L.I | L.II | L.III | L.IV |
|---|---|---|---|---|
| ingredients | Wt-% in formulation | | | |
| n-$C_{18}$-alkyl-$(OCH_2CH_2)_{25}$-OH | - | 4 | 4 | - |
| Tallow oil soap $C_{14}$-$C_{18}$ Carbonic acid, sodium salt | 3 | 2 | 2 | 3 |
| Sodium $C_{10}$-$C_{12}$-alkyl benzenesulfonate | 14 | 14 | 14 | 14 |
| Sodium laurethsulfate - n-$C_{12}H_{25}$-O-$(CH_2CH_2O)_3$-$SO_3Na$ | 4 | 4 | 4 | 4 |
| Complexing agent | 2.0 EDTA | 2.0 EDTA | 2.5 DTPA | 2.5 HEDP |
| mixture Na-citrate:Na-formiate 9:1 | 4 | 4 | 4 | 4 |
| Glycerol | 2.5 | 2 | 2.5 | 2 |
| Ethanol | 1.5 | 1.5 | 1.5 | 1.5 |
| Propyleneglycol | 3 | 3.5 | 3 | 3.5 |
| Additives: | | | | |
| GPM or C-GP according to table Ex-IIb** | 2.5 | 2.5 | 2.0 | 3.0 |
| Protease Lavergy® Pro114LS | 0.8 | 0.8 | 0.8 | |
| Stainzyme® | 0.3 | 0.3 | 0.3 | 0.3 |
| Celluclean® 5000L | 0.2 | 0.2 | 0.2 | 0.2 |
| Mannaway® | 0.2 | 0.1 | 0.2 | 0.2 |
| balance | Water to 100 | | | |
| ** for comparative tests without GPM or C-GP those were replaced by the same amount of water. | | | | |

The primary wash performance of inventive formulations was tested in a washing machine preparing wash solutions using water of 14°dH hardness (2.5 mmol/L; Ca:Mg:$HO_3$ 4:1:8) containing 3.0 g/L of the liquid test detergents L.1 to L.IV, see composition in Table Ex-IIa and 2.0% to 3.0% by weight of the GPM or C-GP as well as the enzyme formulation (see table Ex-IIa).

For the performance test in the washing machine (Miele SOFTTRONIC W 1935 WTL, 30°C, short program, 1200 rpm, 3.5 kg ballast load), three multi-stain monitors (M1, M2, M3) were washed together with four SBL-2004 sheets (wfk Testgewebe GmbH, DE; corresponding to 64 grams of ballast soil) as additional soil ballast.

Pre-soiled test fabrics have been purchased from wfk test fabrics GmbH, Krefeld; EMPA = Swiss Federal Institute of Materials Testing; or CFT = Center for Test Material B.V.

Multi-stain monitors for the washing machine tests:

M1 (8 stains):
PC-H144 Red pottery clay; KC-H115 Standard clay; P-H145 Tennis court clay; KC-H018 Clay, ground soil; EMPA 101 Olive oil/carbon black; CFT C-10 Pigment/oil/milk; W-20D Sebum Bey; E-117 Blood/Milk/Ink.

M2 (12 stains):
wfk20D Sebum bey; EMPA 141/2 Lipstick medium soiled on cotton; CFT PC-S-04 Olive oil, colored; EMPA 112 Cocoa; EMPA 116 Blood/milk/ink; CFT C-10 Pigment/oil/milk; CFT PC-05 Blood/milk/ink; CFT C-S-38 Egg yolk with pigment, aged; CFT C-S-129 Tapioca Starch, aged; W-20D Sebum Bey; EMPA 125 Surfactant Monitor; EMPA 163 Porridge.

M3 (12 stains):
CFT CS-06 Salad dressing; CFT CS-80 Grass/mud; CFT C-05 Blood/milk/ink; EMPA 117 Blood/milk/ink; CFT C-03 Chocolate milk with soot; E-160 Chocolate Cream; CFT C-11 Milk with carbon black; CFT C-S-70 Chocolate mousse; CFT C-S-39 Full egg with pigment, aged; C-S-126 Corn Starch, aged; C-S-127 Potato Starch, aged; C-S-128 Rice Starch, aged.

The total level of cleaning was evaluated using color measurements. Reflectance values of the stains on the monitors were measured using a sphere reflectance spectrometer (SF 500 type from Datacolor, USA, wavelength range 360-700nm, optical geometry d/8°) with a UV cutoff filter at 460 nm. In this case, with the aid of the CIE-Lab color space classification, the brightness L *, the value a * on the red- green color axis and the b * value on the yellow-blue color axis, were measured before and after washing and averaged for the respective stains of the monitor. The change of the color value (Delta E, ΔE) value, defined and calculated automatically by the evaluation color tools on the following formula,

$$\Delta E^*_{ab} = \sqrt{\Delta L^{*2} + \Delta a^{*2} + \Delta b^{*2}},$$

which is a measure of the achieved cleaning effect. All experiments were repeated three times to provide a representative average number. Higher Delta E values show better cleaning. For each stain, a difference of 1 unit can be detected visually by a skilled person. A non-expert can visually detect 2 units easily. The ΔE values of the formulations for the 4,11 and 104 stains of correspondingly M1, M2 and M3 are shown in Table Ex-IIb.

Table Ex-IIb: Results of washing machine test fabric monitors

| Detergent | | ΔE MS1 | ΔE MS2 | ΔE MS3 | Detergent | | ΔE MS1 | ΔE MS2 | ΔE MS3 |
|---|---|---|---|---|---|---|---|---|---|
| See Tabl e Ex-IIa | Graft polymer | | | | Base formu lation | Graft polymer | | | |
| L.I | - | 98.8 | 136.6 | 163.0 | L.II | - | 97.2 | 133.8 | 160.6 |
| L.I | GPM.1a | 117.7 | 168.4 | 195.5 | L.II | GPF.1 | 116.0 | 174.6 | 195.9 |
| L.I | GPF.1 | 119.1 | 173.5 | 197.0 | L.II | GPM.2 | 116.2 | 170.3 | 191.4 |
| L.I | GPM.4 | 114.3 | 164.8 | 191.4 | L.II | GPM.3 | 111.3 | 165.3 | 190.1 |
| L.I | GPM.6 | 112.8 | 166.0 | 192.7 | L.II | GPM.4 | 113.4 | 168.4 | 191.8 |
| L.I | GPM.7 | 110.4 | 161.8 | 190.5 | L.II | GPM.5 | 111.5 | 162.4 | 187.2 |
| L.I | GPM.10 | 114.9 | 163.0 | 192.0 | L.II | GPM.10 | 114.9 | 166.0 | 189.3 |
| L.I | GPM.11 | 112.6 | 167.5 | 193.5 | L.II | GPM.12 | 112.0 | 168.5 | 192.0 |
| L.I | GPM.12 | 114.0 | 165.0 | 191.6 | L.II | GPM.14 | 115.0 | 164.0 | 190.8 |

(continued)

| Detergent | | ΔE MS1 | ΔE MS2 | ΔE MS3 | Detergent | | ΔE MS1 | ΔE MS2 | ΔE MS3 |
|---|---|---|---|---|---|---|---|---|---|
| See Tabl e Ex-IIa | Graft polymer | | | | Base formu lation | Graft polymer | | | |
| L.I | GPM.14 | 111.3 | 162.0 | 189.6 | L.II | GPM.15 | 111.9 | 167.5 | 193.2 |
| L.I | C-GP. 17 | 104.6 | 144.0 | 171.5 | L.II | C-GP. 17 | 102.4 | 142.5 | 172.3 |
| L.I | C-GP. 18 | 106.0 | 146.5 | 170.0 | L.II | C-GP. 18 | 104.7 | 145.0 | 170.9 |
| | | | | | | | | | |
| L.III | - | 100.6 | 140.4 | 165.0 | L.IV | GPM.1a | 121.5 | 166.8 | 192.6 |
| L.III | GPM.1 a | 120.0 | 167.5 | 189.3 | L.IV | GPM.2 | 119.5 | 164.6 | 191.0 |
| L.III | GPM.2 | 117.4 | 163.7 | 187.6 | L.IV | GPM.3 | 116.4 | 162.1 | 188.7 |
| L.III | GPM.3 | 113.5 | 158.8 | 185.6 | L.IV | GPM.4 | 117.5 | 165.0 | 190.0 |
| L.III | GPM.5 | 112.0 | 162.7 | 185.2 | L.IV | GPM.5 | 115.6 | 163.0 | 187.9 |
| L.III | GPM.10 | 115.4 | 165.0 | 189.0 | L.IV | GPM.10 | 117.5 | 161.9 | 188.3 |
| L.III | GPM.11 | 114.8 | 162.8 | 187.3 | L.IV | GPM.12 | 114.8 | 162.4 | 190.5 |
| L.III | GPM.13 | 110.7 | 157.5 | 183.5 | L.IV | GPM.14 | 116.6 | 163.5 | 192.0 |
| L.III | GPM.14 | 115.6 | 164.6 | 188.6 | L.IV | - | 100.8 | 141.8 | 163.8 |
| L.III | B16 | 119,6 | 166,6 | 1878, 6 | L.IV | - | 99,1 | 142,3 | 163 |
| L.III | C-GP. 17 | 107.0 | 147.3 | 172.6 | L.IV | C-GP. 17 | 106.8 | 149.5 | 172.1 |
| L.III | C-GP. 18 | 105.7 | 149.5 | 1713. 4 | L.IV | C-GP. 18 | 108.0 | 150.9 | 171.4 |

The above tests demonstrate a high-performance level in the presence of complexing agents after a significant time of storage. The enzymes are obviously protected by the inventive polymer formulation and consequently show a full fresh performance. Despite no lipase is present, the liquid detergent is most surprisingly very powerful against fatty stains. This is especially beneficial, and we can confirm the absence of malodor which is the bad side of a lipase.

III. dish washing experiments

The base detergent composition according to Table Ex-IIIa was used for making liquid dish (ADW) detergent compositions according to the invention and comparison detergent compositions.

Table Ex-IIIa: liquid detergent formulation

| Dish washing formulation: | DW.I | DW.II | DW.III | DW.IV |
|---|---|---|---|---|
| Ingredients | wt% in formulation | | | |
| Trilon M fl (Max liqu); 50% solution of MGDA in water | 20 | 15 | 10 | - |
| Citric acid | - | 5 | - | 20 |
| GLDA 50% solution in water | - | - | 15 | 10 |
| Polyacrylic acid Mw 5.000 g/mol | 5 | 2.5 | 5 | 5 |
| Glycerol (G) or Propanediol (P) | 5 G | 5 P | 5 G | 5 P |

(continued)

| Dish washing formulation: | DW.I | DW.II | DW.III | DW.IV |
|---|---|---|---|---|
| Ingredients | wt% in formulation | | | |
| NIS* | 2.5 | 2.0 | 2.5 | 2.5 |
| $Na_4HEDP$ | - | 1 | - | 2 |
| Xanthan gum | 0.3 | 0.3 | 0.3 | 0.3 |
| Additives: | | | | |
| Protease | 3.5 Lavergy 104LS | 3.5 Lavergy 104LS | 3.5 Lavergy 114LS | 3.5 Lavergy 114LS |
| Amplify ® Prime 100L | 0.5 | 0.5 | 0.5 | 0.5 |
| GPM or C-GP | 2.5 | 3.0 | 3.0 | 2.5 |
| balance | Water to 100 after adjust pH 8.0 with citric acid | | | |
| * NIS according to formula NIS-I as disclosed herein, wherein $R^1$ is n-$C_8$ alkyl, $R^2$ is branched $C_{11}$ alkyl, AO is $CH_2$-$CH_2$-O, and x is 22. | | | | |

The liquid detergent formulations were stored at a temperature of 37° for 6 weeks (42 days) before performance tests were executed.

Spotting, filming, fat residue and odor tests:

All dish-wash experiments were carried out in Miele automatic dish wash machines, type G1222 SCL. The program 40°C ("R-time 2", for washing) and 55° for rinsing was selected. No separate rinsing agent was added, no regenerating salt was used. Before the tests started the sieves of the dishwashers were weighed. The dish-wash experiments were carried out with water, 21° dH (German hardness), Ca/Mg:$HCO_3$ (3:1):1.35. In each experiment three knives (stainless steel), three blue melamine resin plates, eleven drinking glasses and fourteen plates from china were placed in the dishwasher. Before each cycle, 5 g of Biskin Gold®, a solid vegetable fat, and 5 g of margarine and 50 g additional ballast soil (RA mix) were added. In each cycle of an experiment according to the invention, 18 g of a detergent composition according to table 3 were added.

5 cycles were run without drying times between cycles. After the 5$^{th}$ cycles odor was noted by three test persons and the sieve was taken out of the machine for inspection and evaluation-then again put into the machine which was closed and stored for another 48 hours at 25°C. Then weights of the sieves (fat) were determined and the differences to their weights before the first cycle were calculated. Finally, visual assessment with respect to filming and soiling were executed on porcelain, glass, cutlery and plastic individually and on the average. The visual assessment was made according to the following table rank:

- 7 points: excellent; without any marks, no filming
- 5-6 points: good, 1 to 4 spots almost invisible filming
- 2-4 points: fair, more than 4 spots to 25% of the surface covered with stains, moderate filming
- 1 point: 25 to 50% of the surface covered with stains
- 0 points: almost completely covered with spots, significant filming

In table Ex-IIIb, only the average values are shown (values with number round and 0.5 as possible fine scale).

Odor is ranked in 4 grades: from 4 = neutral, no odor, to 1= significant malodor (fool, fishy); value is average of 3 evaluators: The results are summarized in Table Ex-IIIb.

Table Ex-IIIb: Automatic dishwashing tests

| Detergent | | Spots Average | Filming Average | Fat in sieve [g] | Cleaning Performanc e | Odor 48h 25°C After 5th cycle |
|---|---|---|---|---|---|---|
| Formulation according to Table Ex-IIIa | GPM | | | | | |
| DW.I | GPF.1 | 25 | 26.6 | 2.3 | 42 | 3 |
| DW.I | GPM. 3 | 26.5 | 25 | 2.9 | 36 | 2.5 |
| DW.I | GPM. 8 | 27 | 28 | 2.4 | 38 | 2.5 |
| DW.I | GPF.8 | 29.5 | 24 | 2.0 | 43 | 3.5 |
| DW.I | GPM. 9 | 28.5 | 27 | 2.8 | 38 | 3 |
| DW.I | GPF.9 | 31 | 30 | 2.1 | 37 | 3 |
| DW.I | - | 21 | 18.5 | 3.8 | 21 | 1.5 |
| DW.I | C-GP. 17 | 22.5 | 20.0 | 3.6 | 24 | 1.5 |
| | | | | | | |
| DW.II | GPF.1 | 25 | 27 | 2.6 | 40 | 2.5 |
| DW.II | GPM. 3 | 27 | 26 | 3.0 | 34 | 2.5 |
| DW.II | GPM. 8 | 26 | 27 | 2.9 | 36 | 2.5 |
| DW.II | GPF.8 | 30 | 32 | 2.3 | 38 | 3 |
| DW.II | GPF.9 | 28.5 | 30 | 1.9 | 39 | 3.5 |
| DW.II | GPM. 13 | 28 | 27 | 2.9 | 35 | 3.5 |
| DW.II | - | 20 | 17 | 4.0 | 19 | 1.5 |
| DW.II | C-GP. 17 | 21 | 21 | 3.7 | 24 | 1.5 |
| | | | | | | |
| DW.III | GPF.1 | 24.5 | 24 | 2.5 | 39 | 3.5 |
| DW.III | GPM. 8 | 26 | 26 | 2.6 | 35 | 3 |
| DW.III | GPF.8 | 29 | 30 | 2.1 | 39 | 2.5 |
| DW.III | GPM. 13 | 28 | 30 | 2.5 | 40 | 3 |
| DW.III | GPM. 12 | 26 | 27 | 2.3 | 37 | 3 |
| DW.III | GPF. 12 | 27.5 | 26 | 1.9 | 39 | 3.5 |
| DW.III | - | 19 | 18 | 3.4 | 23 | 2 |
| DW.III | C-GP. 17 | 20 | 21 | 3.1 | 25 | 2 |
| | | | | | | |

(continued)

| Detergent | | Spots Average | Filming Average | Fat in sieve [g] | Cleaning Performanc e | Odor 48h 25°C After 5$^{th}$ cycle |
|---|---|---|---|---|---|---|
| Formulation according to Table Ex-IIIa | GPM | | | | | |
| DW.IV | GPF1 | 25.5 | 24 | 2.5 | 36 | 3 |
| DW.IV | GPM. 3 | 26 | 27 | 2.1 | 33 | 2.5 |
| DW.IV | GPF.8 | 29.5 | 28 | 2.6 | 36 | 3 |
| DW.IV | GPF.9 | 28 | 26.5 | 2.3 | 37 | 3 |
| DW.IV | GPM. 12 | 29 | 27 | 2.4 | 34 | 3 |
| DW.IV | GPF. 12 | 32 | 28 | 2.1 | 41 | 3.5 |
| DW.IV | - | 21 | 18.5 | 3.6 | 18 | 2 |
| DW.IV | C-GP. 17 | 23 | 20.5 | 3.2 | 22 | 2 |

Cleaning tests were executed as described by German Cosmetic, Toiletry, Perfumery and Detergent Association (IKW); sofw Journal, 06/16), Volume 142, p 34-48 (Recommendations for the Quality Assessment of the Cleaning Performance of Dishwasher Detergents; Part B);
Machine: Miele G123 SCL, program 45°C; water hardness 21°dH; ballast soil: 50g IKW soil, machine load: glass, china, plastic crockery; stained teacups, egg yolk on steel; DM 277 (mixed starch); cycles: 2 conditioning, 2 cleaning

The results of evaluation in accordance to the IKW photo catalogue (unwashed =0; 1-10) are shown in table 5. The values represent the sum of 5 cleaning test experiments/cycles (max. 50).

The addition of inventive graft copolymer formulation (GPF) significantly reduced the fat residue and improve the cleaning performance in the test and also improve machine hygiene by limiting malodor.

The combined action solves the issues with respect to the task to improve cleaning performance at 45°C ADW with low odor and at the same time the fatty residues in the machine are kept low.

## Claims

1. Mixture comprising at least

component (a): graft polymer comprising as a graft base a polyether and as grafted side chains copolymers comprising at least one comonomer selected from

$CH_2$=CH-O-C(O)-$R^3$ (Ia)

$CH_2$=CH-$CH_2$-O-C(O)-$R^3$ (Ib)

$CH_2$=CZ-CO-$OR^4$ (Ic)

wherein $R^3$ is selected from $C_1$-$C_{21}$-alkyl, for example methyl, n-propyl, n-pentyl, n-heptyl, n-nonyl, iso-nonyl, n-undecyl, n-tridecyl, n-pentadecyl, n-heptadecyl, or n-nonadecyl.
$R^4$ is selected from $C_2$-$C_{20}$-alkyl, preferably with an even number of carbon atoms, for example ethyl, n- and iso propyl, n-hexyl, isohexyl, sec-hexyl, n-heptyl, n-octyl, 2-ethylhexyl, n-nonyl, n-decyl or isodecyl, n-$C_{12}H_{25}$, n-$C_{14}H_{29}$, n-$C_{16}H_{33}$ or n-$C_{18}H_{37}$,
and Z is selected from hydrogen and methyl, hydrogen bring preferred
component (b): Trialkyl citrate according to formula (TAC):

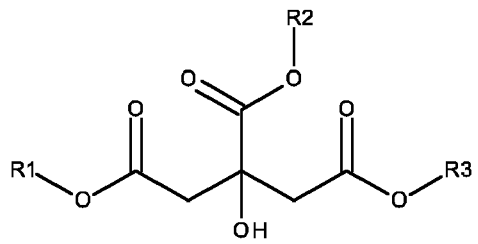

(TAC)

wherein the variables in formula (TAC) are defined as follows:
R1, R2, R3 are selected from H, linear C1-C8 alkyl, and branched C3-C8 alkyl, wherein at least one of R1, R2, and R3 is not H. Examples of linear C1-C8 alkyl are methyl, ethyl, n-propyl, n-butyl, n-pentyl, etc.
Examples of branched C3-C8 alkyl are 2-propyl, 2-butyl, sec.-butyl, tert.-butyl, 2-pentyl, 3-pentyl, iso-pentyl, etc. Preferably, R1, R2, R3 are ethyl.

2. Mixture according to claim 1, which results from addition of at least one trialkylcitrate according to formula (TAC)

(a) during the grafting process to produce GP or
(b) before or during the step of removal of volatile components from GP or
(c) during or after the cooling step following the removal of volatile components.

3. Mixture according to any preceding claim, wherein the weight ratio of GP:TAC is within the range of 10:1 to 1:1.

4. Mixture according to any preceding claim additionally comprising at least one surfactant selected from non-ionic surfactants (NIS) and amphoteric surfactants (AS).

5. Method of preparing the graft polymer mixture according to claims 1-3, by the steps of

i. heating at least one polyether as disclosed above to about 50-120°C, preferably about 70-100°C, more preferably about 80-95°C, such as about 90°C under $N_2$-atmosphere, at reduced, ambient or increased pressure, preferably ambient pressure

ii. feeding radical starter (suitable for the chosen reaction conditions) over a time period of about 3-10h, preferably about 5-9h, more preferably about 6-8h, such as about 7h (feed 1)

iii. 0-30 min, preferably 5-20 min, such as 15 min +/-2 min after feed 1 commenced, feeding at least one comonomer (CM) as disclosed above over a time period of about 3-8h, preferably about 4-7h, more preferably about 5-6h (feed 2)

iv. after completion of feed 2, feeding radical starter over a time period of about 0.5-5h, preferably about 1-4h, more preferably about 2-3h (feed 3)

v. after completion of feed 3, stirring at a temperature in the range of 100°C to 130°C for a time period of about 60min to 90 min

vi. reducing pressure to 10 to 40 mbar for removal of volatile components

vii. cooling the reaction mixture,

wherein at least one trialkyl citrate is added during or directly after step (ii), during or directly after step (iii), during or directly after step (v.) or during or directly after step (vii.).

**6.** Method of preparing the mixture according to claim 4 by adding at least one non-ionic surfactant and/or at least one amphoteric surfactant to a mixture according to claims 1-3.

**7.** Liquid detergent formulation comprising the mixture according to claims 1-4, at least one hydrolase (EC.3) and at least one detergent component selected from surfactants, complexing agents and solvents.

**8.** Use of mixture according to claims 1-4 for stabilizing at least one hydrolase within a liquid detergent formulation.

**9.** Use of a detergent formulation according to claim 7 to increase wash performance after storage of the detergent formulation at 37°C for up to 42 days when compared to a detergent formulation stored under the same storing condition but lacking the mixture according to claims 1-4.

**10.** Use according to claims 9, wherein the fat-removal activity of the detergent formulation is increased when compared to using detergent formulations lacking the mixture according to claims 1-4.

Europäisches Patentamt
European Patent Office
Office européen des brevets

# EUROPEAN SEARCH REPORT

Application Number
EP 20 21 5594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/069913 A1 (BASF SE [DE]) 9 April 2020 (2020-04-09) * claims 1, 5 * * examples on pages 45-50 * * tables 1-4 * ----- | 1-10 | INV. C12N9/96 |
| Y | US 5 082 585 A (HESSEL JOHN F [US] ET AL) 21 January 1992 (1992-01-21) * example IV * * table 1 * * column 2, line 4 - line 31 * ----- | 1-4,7-10 | |
| A | GB 922 457 A (HOECHST AG) 3 April 1963 (1963-04-03) * example 1 * ----- | 1-10 | |
| Y | WO 2019/197315 A1 (BASF SE [DE]) 17 October 2019 (2019-10-17) * example 1.6 on page 2 * ----- | 5,6 | |

TECHNICAL FIELDS SEARCHED (IPC)

C11D
C12N

The present search report has been drawn up for all claims

1

| Place of search | Date of completion of the search | Examiner |
|---|---|---|
| The Hague | 31 May 2021 | Placke, Daniel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 21 5594

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2021

| Patent document cited in search report | Publication date | Patent family member(s) | Publication date |
|---|---|---|---|
| WO 2020069913 A1 | 09-04-2020 | CN 112805376 A | 14-05-2021 |
| | | WO 2020069913 A1 | 09-04-2020 |
| US 5082585 A | 21-01-1992 | NONE | |
| GB 922457 A | 03-04-1963 | FR 1222944 A | 14-06-1960 |
| | | GB 922457 A | 03-04-1963 |
| WO 2019197315 A1 | 17-10-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0018375 A
- EP 283075 A2
- WO 8906279 A
- WO 9102792 A
- WO 9523221 A
- DE 10064983
- WO 2003054184 A
- WO 2003056017 A
- WO 2003055974 A
- WO 2005063974 A
- WO 2005103244 A
- DE 102005028295
- EP 1921147 A
- US 5869438 A
- WO 9510603 A
- WO 9402597 A
- WO 94018314 A
- WO 97043424 A
- WO 99019467 A
- WO 0210355 A
- WO 9919467 A
- WO 9623872 A
- WO 973296 A
- WO 99194671 A
- WO 2013001078 A
- WO 0022103 A
- WO 2009061380 A
- WO 2013184577 A
- WO 2010104675 A
- WO 0060060 A
- WO 2016092009 A
- WO 2006002643 A
- WO 2011098531 A
- WO 2013001087 A
- WO 2006066594 A
- WO 2014183920 A
- WO 2014183921 A
- WO 2018224544 A
- WO 2004053039 A
- WO 2018184767 A
- WO 2018220274 A
- WO 2009074685 A
- WO 9324622 A
- WO 2008009673 A
- WO 2005003319 A
- WO 2019081724 A
- WO 2019081721 A
- WO 2017060493 A
- WO 9117244 A


### Non-patent literature cited in the description


- **NEEDLEMAN ; WUNSCH.** *J. Mol. Biol.,* 1979, vol. 48, 443-453
- **VASANTHA E.** *J. Bacteriol,* 1984, vol. 159, 811-819
- **JA WELLS et al.** *Nucleic Acids Research,* 1983, vol. 11, 7911-7925
- **EL SMITH et al.** *J. Biol Chem,* 1968, vol. 243, 2184-2191
- **JACOBS et al.** *Nucl. Acids Res,* 1985, vol. 13, 8913-8926
- **GUPTA et al.** *Appl. Microbiol. Biotechnol,* 2002, vol. 60, 381-395
- **DELMAR et al.** *Analytical Biochem,* 1979, vol. 99, 316-320
- **GUPTA et al.** *Biotechnol. Appl. Biochem.,* 2003, vol. 37, 63-71
- **HOFFMAN, W. S.** *J. Biol. Chem.,* 1937, vol. 120, 51
- **MCCLEARY, B. V.** *Carbohydrate Research,* 1978, vol. 67 (1), 213-221
- **MILLER, G. L.** Use of Dinitrosalicylic Acid Reagent for Determination of Reducing Sugars. *Analytical Chemistry,* 1959, vol. 31, 426-428
- Recommendations for the Quality Assessment of the Cleaning Performance of Dishwasher Detergents; Part B. sofw Journal. German Cosmetic, Toiletry, Perfumery and Detergent Association (IKW), June 2016, vol. 142, 34-48